# EUROPEAN PATENT SPECIFICATION

(11) **EP 1 210 344 B1**
(45) Date of publication and mention of the grant of the patent: **19.10.2005**
(21) Application number: 00955657.2
(22) Date of filing: 18.08.2000
(51) Int. Cl.: C07D 413/04, C07D 401/04, A61K 31/4439, A61K 31/4725, A61K 31/497, A61P 25/00

(54) **HETEROPOLYCYCLIC COMPOUNDS AND THEIR USE AS METABOTROPIC GLUTAMATE RECEPTOR ANTAGONISTS**
HETEROPOLYCYCLISCHE VERBINDUNGEN UND IHRE VERWENDUNG ALS ANTAGONISTEN VON METABOTROPEN GLUTAMATREZEPTOREN
COMPOSES HETEROPOLYCYCLIQUES ET LEUR UTILISATION EN TANT QU'ANTAGONISTES DES RECEPTEURS DU GLUTAMATE METABOTROPE

(30) Priority: 19.08.1999 US 149464 P
(43) Date of publication of application: 05.06.2002
(60) Divisional application: 05014788.3
(73) Proprietor: NPS PHARMACEUTICALS, INC., Salt Lake City Utah 84108 (US)
(72) Inventor: VAN WAGENEN, Bradford, C., Salt Lake City, UT 84127 (US); STORMANN, Thomas, M., Salt lake City, UT 84105 (US); MOE, Scott, T., Salt Lake City, UT 84121 (US); SHEEHAN, Susan, M., Salt Lake City, UT 84108 (US); MCLEOD, Donald, A., Salt Lake City, UT 84121 (US); SMITH, Daryl, L., Salt Lake City, UT 84121 (US); ISAAC, Methvin, Benjamin, Etobicoke, Ontario M9V 5E3 (CA); SLASSI, Abdelmalik, Mississauga, Ontario L5N 5G4 (CA)
(74) Representative: Plougmann & Vingtoft A/S
(86) International application number: PCT/US2000/022618
(87) International publication number: WO 2001/012627

(56) References cited:
- WO-A-94/22846
- WO-A-97/03967
- WO-A-98/17652
- WO-A-99/26927
- US-A- 3 647 809
- US-A- 4 022 901
- PERRIER H ET AL: "Substituted furans as inhibitors of the PDE4 enzyme" BIOORGANIC & MEDICINAL CHEMISTRY LETTERS, vol. 9, no. 3, 8 February 1999 (1999-02-08), pages 323-326, XP004157222 ISSN: 0960-894X
- KELLY T R ET AL: "Total synthesis of dimethyl sulfomycinamate" TETRAHEDRON LETTERS, vol. 36, no. 30, 24 July 1995 (1995-07-24), pages 5319-5322, XP004027670 ISSN: 0040-4039
- MASSA M A ET AL: "Synthesis of novel substituted pyridines as inhibitors of endothelin converting enzyme-1 (ECE-1)" BIOORGANIC & MEDICINAL CHEMISTRY LETTERS, vol. 8, no. 16, 18 August 1998 (1998-08-18), pages 2117-2122, XP004137230 ISSN: 0960-894X
- PAUDLER W W ET AL: "The conversion of imidazo[1,5-a]pyridines into 3-(2-pyridyl)-1,2,4-oxadiazoles" JOURNAL OF ORGANIC CHEMISTRY, vol. 32, no. 8, 11 August 1967 (1967-08-11), pages 2430-2433, XP002153136
- DATABASE CROSSFIRE [Online] Beilstein Institut zur Foerderung der Chemischen Wissenschaf; XP002153140 & ARCH. PHARM., vol. 321, 1988, pages 713-717,
- HUANG W ET AL: "A novel improved procedure for the synthesis of oxazoles" TETRAHEDRON, vol. 52, no. 30, 22 July 1996 (1996-07-22), pages 10131-10136, XP002153137
- PEI W ET AL: "Convenient syntheses of 2-alkyl(aryl)-4,5-diphenyloxazoles and 2-alkyl(aryl)-4-phenyloxazoles" SYNTHESIS, no. 9, September 1998 (1998-09), pages 1298-1304, XP002153138
- OHKUBO M ET AL: "Studies on cerebral protective agents. VII. Synthesis of novel 4-arylazole derivatives with anti-anoxic activity" CHEMICAL & PHARMACEUTICAL BULLETIN, vol. 43, no. 6, June 1995 (1995-06), pages 947-954, XP002153139

## Description

### FIELD OF THE INVENTION

The invention provides compounds active at metabotropic glutamate receptors and that are useful for treating neurological and psychiatric diseases and disorders.

### BACKGROUND OF THE INVENTION

Recent advances in the elucidation of the neurophysiological roles of metabotropic glutamate receptors have established these receptors as promising drug targets in the therapy of acute and chronic neurological and psychiatric disorders and diseases. However, the major challenge to the realization of this promise has been the development of metabotropic glutamate receptor subtype-selective compounds.

Glutamate is the major excitatory neurotransmitter in the mammalian central nervous system (CNS). Glutamate produces its effects on central neurons by binding to and thereby activating cell surface receptors. These receptors have been divided into two major classes, the ionotropic and metabotropic glutamate receptors, based on the structural features of the receptor proteins, the means by which the receptors transduce signals into the cell, and pharmacological profiles.

The metabotropic glutamate receptors (mGluRs) are G protein-coupled receptors that activate a variety of intracellular second messenger systems following the binding of glutamate. Activation of mGluRs in intact mammalian neurons elicits one or more of the following responses: activation of phospholipase C; increases in phosphoinositide (PI) hydrolysis; intracellular calcium release; activation of phospholipase D; activation or inhibition of adenyl cyclase; increases or decreases in the formation of cyclic adenosine monophosphate (cAMP); activation of guanylyl cyclase; increases in the formation of cyclic guanosine monophosphate (cGMP); activation of phospholipase A₂; increases in arachidonic acid release; and increases or decreases in the activity of voltage- and ligand-gated ion channels. Schoepp *et al*., *Trends Pharmacol. Sci. 14*:13 (1993); Schoepp, *Neurochem. Int. 24*:439 (1994); Pin *et al*., *Neuropharmacology 34*:1 (1995).

Eight distinct mGluR subtypes, termed mGluR1 through mGluR8, have been identified by molecular cloning. *See, for example,* Nakanishi, *Neuron 13*:1031 (1994); Pin *et al*., *Neuropharmacology 34*:1 (1995); Knopfel *et al*., *J. Med. Chem. 38*:1417 (1995). Further receptor diversity occurs via expression of alternatively spliced forms of certain mGluR subtypes. Pin *et al*., *PNAS 89*:10331 (1992); Minakami *et al*., *BBRC 199*:1136 (1994); Joly *et al*., *J. Neurosci. 15*:3970 (1995).

Metabotropic glutamate receptor subtypes may be subdivided into three groups, Group I, Group II, and Group III mGluRs, based on amino acid sequence homology, the second messenger systems utilized by the receptors, and by their pharmacological characteristics. Nakanishi, *Neuron 13*:1031 (1994); Pin *et al*., *Neuropharmacology 34*:1 (1995); Knopfel *et al*., *J. Med. Chem. 38*:1417 (1995).

Group I mGluRs comprise mGluR1, mGluR5, and their alternatively spliced variants. The binding of agonists to these receptors results in the activation of phospholipase C and the subsequent mobilization of intracellular calcium. Electrophysiological measurements have been used to demonstrate these effects, for example, in *Xenopus* oocytes that express recombinant mGluR1 receptors. *See, for example,* Masu *et al*., *Nature 349*:760 (1991); Pin *et al*., *PNAS 89*:10331 (1992). Similar results have been achieved with oocytes expressing recombinant mGluR5 receptors. Abe *et al*., *J. Biol. Chem. 267*:13361 (1992); Minakami *et al*., *BBRC 199*:1136 (1994); Joly *et al., J. Neurosci. 15*:3970 (1995). Alternatively, agonist activation of recombinant mGluR1 receptors expressed in Chinese hamster ovary (CHO) cells stimulates PI hydrolysis, cAMP formation, and arachidonic acid release as measured by standard biochemical assays. *Aramori et al., Neuron 8*:757 (1992).

By comparison, the activation of mGluR5 receptors, expressed in CHO cells, stimulates PI hydrolysis and subsequent intracellular calcium transients, but no stimulation of cAMP formation or arachidonic acid release is observed. Abe *et al*., *J. Biol. Chem*. *267*:13361 (1992). However, activation of mGluR5 receptors expressed in LLC-PK1 cells results in PI hydrolysis and increased cAMP formation. Joly *et al*., *J. Neurosci. 15*:3970 (1995). The agonist potency profile for Group I mGluRs is quisqualate > glutamate = ibotenate > *(2S*,*1'S*,*2'S)*-2-carboxycyclopropyl)glycine (L-CCG-I) > *(1S*,*3R)*-1-aminocyclopentane-1,3-dicarboxylic acid (ACPD). Quisqualate is relatively selective for Group I receptors, as compared to Group II and Group III mGluRs, but it also is a potent activator of ionotropic AMPA receptors. Pin *et al*., *Neuropharmacology 34*:1, Knopfel *et al*., *J. Med. Chem. 38*:1417 (1995).

The lack of subtype-specific mGluR agonists and antagonists has impeded elucidation of the physiological roles of particular mGluRs, and the mGluR-associated pathophysiological processes that affect the CNS have yet to be defined. However, work with the available non-specific agonists and antagonists has yielded some general insights about the Group I mGluRs as compared to the Group II and Group III mGluRs.

Attempts at elucidating the physiological roles of Group I mGluRs suggest that activation of these receptors elicits neuronal excitation. Various studies have demonstrated that ACPD can produce postsynaptic excitation upon application to neurons in the hippocampus, cerebral cortex, cerebellum, and thalamus, as well as other brain regions. Evidence indicates that this excitation is due to direct activation of postsynaptic mGluRs, but it also has been suggested that activation of presynaptic mGluRs occurs, resulting in increased neurotransmitter release. Baskys, *Trends Pharmacol. Sci. 15*:92 (1992); Schoepp, *Neurochem. Int. 24*:439 (1994); Pin *et al., Neuropharmacology 34*:1(1995).

Pharmacological experiments implicate Group I mGluRs as the mediators of this excitatory mechanism. The effects of ACPD can be reproduced by low concentrations of quisqualate in the presence of iGluR antagonists. Hu *et al*., *Brain Res. 568*:339 (1991); *Greene et al., Eur. J. Pharmacol. 226*:279 (1992). Two phenylglycine compounds known to activate mGluR1, namely *(S)*-3-hydroxyphenylglycine (*(S)*-3HPG) and *(S)*-3,5-dihydroxyphenylglycine (*(S)*-DHPG), also produce excitation. Watkins *et al*., *Trends Pharmacol. Sci. 15*:33 (1994). In addition, the excitation can be blocked by *(S)-4*-carboxyphenylglycine (*(S)*-4CPG), *(S)*-4-carboxy-3-hydroxyphenylglycine (*(S)*-4C3HPG), and (+)-alpha-methyl-4-carboxyphenylglycine ((+)-MCPG), compounds known to be mGluR1-antagonists. Eaton *et al*., *Eur. J. Pharmacol. 244*:195 (1993); Watkins *et al*., *Trends Pharmacol. Sci. 15*:333 (1994).

Metabotropic glutamate receptors have been implicated in a number of normal processes in the mammalian CNS. Activation of mGluRs has been shown to be required for induction of hippocampal long-term potentiation and cerebellar long-term depression. *Bashir et al*., *Nature 363*:347 (1993); Bortolotto *et al., Nature 368*:740 (1994); Aiba *et al*., *Cell 79*:365 (1994); Aiba *et al*., *Cell 79*:377 (1994). A role for mGluR activation in nociception and analgesia also has been demonstrated. Meller *et al., Neuroreport 4:* 879 (1993). In addition, mGluR activation has been suggested to play a modulatory role in a variety of other normal processes including synaptic transmission, neuronal development, apoptotic neuronal death, synaptic plasticity, spatial learning, olfactory memory, central control of cardiac activity, waking, motor control, and control of the vestibulo-ocular reflex. Generally, see Nakanishi, *Neuron 13*: 1031 (1994); Pin *et al*., *Neuropharmacology 34*:1; Knopfel *et al*., *J. Med. Chem. 38*:1417 (1995).

Metabotropic glutamate receptors also have been suggested to play roles in a variety of pathophysiological processes and disease states affecting the CNS. These include stroke, head trauma, anoxic and ischemic injuries, hypoglycemia, epilepsy, and neurodegenerative diseases such as Alzheimer's disease. Schoepp *et al*., *Trends Pharmacol. Sci. 14*:13 (1993); Cunningham *et al., Life Sci. 54*:135 (1994); Hollman *et al., Ann. Rev. Neurosci. 17*:31 (1994); *Pin et al*., *Neuropharmacology 34*:1 (1995); Knopfel *et al*., *J. Med. Chem. 38*:1417 (1995). Much of the pathology in these conditions is thought to be due to excessive glutamate-induced excitation of CNS neurons. Because Group I mGluRs appear to increase glutamate-mediated neuronal excitation via postsynaptic mechanisms and enhanced presynaptic glutamate release, their activation probably contributes to the pathology. Accordingly, selective antagonists of Group I mGluR receptors could be therapeutically beneficial, specifically as neuroprotective agents, analgesics, or anticonvulsants.

Preliminary studies assessing therapeutic potentials with the available mGluR agonists and antagonists have yielded seemingly contradictory results. For example, it has been reported that application of ACPD onto hippocampal neurons leads to seizures and neuronal damage (Sacaan *et al*., *Neurosci. Lett. 139*:77 (1992); Lipparti *et al*., *Life Sci. 52*:85 (1993). Other studies indicate, however, that ACPD inhibits epileptiform activity, and also can exhibit neuroprotective properties. Taschenberger *et al., Neuroreport 3*:629 (1992); Sheardown, *Neuroreport 3*:916 (1992); Koh *et al., Proc. Natl. Acad. Sci. USA 88*:9431 (1991); Chiamulera *et al*., *Eur. J. Pharmacol. 216*:335 (1992); Siliprandi *et al., Eur. J. Pharmacol. 219*:173 (1992); Pizzi *et al*., *J. Neurochem. 61*:683 (1993).

It is likely that these conflicting results are due to the lack of selectivity of ACPD, which causes activation of several different mGluR subtypes. In the studies finding neuronal damage it appears that Group I mGluRs were activated, thereby enhancing undesirable excitatory neurotransmission. In the studies showing neuroprotective effects it appears that activation of Group II and/or Group III mGluRs occurred, inhibiting presynaptic glutamate release, and diminishing excitatory neurotransmission.

This interpretation is consistent with the observation that *(S)*-4C3HPG, a Group I mGluR antagonist and Group II mGluR agonist, protects against audiogenic seizures in DBA/2 mice, while the Group II mGluR selective agonists DCG-IV and L-CCG-I protect neurons from NMDA- and KA-induced toxicity. Thomsen *et al*., *J. Neurochem. 62*:2492 (1994); Bruno *et al*., *Eur. J. Pharmacol. 256*:109 (1994); Pizzi *et al., J. Neurochem. 61*:683 (1993).

Based on the foregoing, it is clear that currently available mGluR agonists and antagonists have limited value, due to their lack of potency and selectivity. In addition, most currently available compounds are amino acids or amino acid derivatives that have limited bioavailabilities, thereby hampering *in vivo* studies to assess mGluR physiology, pharmacology and their therapeutic potential. Compounds that selectively inhibit activation of metabotropic glutamate receptor Group I subtypes should be useful for treatment of neurological disorders and diseases such as senile dementia, Parkinson's disease, Alzheimer's disease, Huntington's Chorea, pain, migraine headaches, epilepsy, head trauma, anoxic and ischemic injuries, psychiatric disorders such as schizophrenia, depression, and anxiety, ophthalmological disorders such as various retinopathies, for example, diabetic retinopathies, glaucoma, and neurological disorders of a auditory nature such as tinnitus, and neuropathic pain disorders, including neuropathic diseases states such as diabetic neuropathies, chemotherapy induced neuropathies, post-herpetic neuralgia, and trigeminal neuralgia.

Accordingly, a need exists for potent mGluR agonists and antagonists that display a high selectivity for a mGluR subtype, particularly a Group I receptor subtype.

### SUMMARY OF THE INVENTION

It is an object of the present invention, therefore, to identify metabotopic glutamate receptor-active compounds which exhibit a high degree of potency and selectivity for individual metabotropic glutamate receptor subtypes, and to provide methods of making these compounds.

It is a further object of this invention to provide pharmaceutical compositions containing compounds which exhibit a high degree of potency and selectivity for individual metabotropic glutamate receptor subtypes, and to provide methods of making these pharmaceutical compositions.

It is yet another object of this invention to provide methods of inhibiting activation of an mGluR Group I receptor, and of inhibiting neuronal damage caused by excitatory activation of an mGluR Group I receptor, specifically mGluR5.

It is still another object of the invention to provide methods of treating a disease associated with excitatory activation of an mGluR Group I receptor, specifically mGluR5.

To accomplish these and other objectives, the present invention provides potent antagonists of Group I mGluRs, specifically, mGluR5. These antagonists are selected from
a) a compound of Formula II: 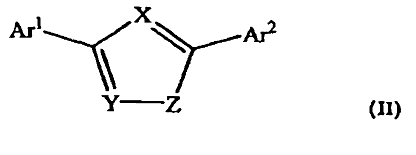 wherein X and Y are N,
   ZisO;
   Ar¹ is 2-pyridyl and Ar² is phenyl, wherein at least one of the Ar¹ and Ar² moieties are substituted with one or more moieties selected from the group consisting of -F, -Cl, -Br, -I, -SR, -SOR, -SO₂R, -SO₂NRR', -OCOR, -OCONRR', -NRCOR', -NRCO₂R', -CN, -CO₂R, - CONRR', -C(O)R, -CH(OR)R', -CH₂(OR), and -R;
   wherein R or R' is selected from the group consisting of H, CF₃, C₁-C₁₀ alkyl, cycloalkyl, alkyl-aryl, heterocycloalkyl, aryl and where R and R' may combine to form a ring,
   with the proviso that the compound is not 3-(2-pyridyl)-5-(2-chlorophenyl)-1,2,4-oxadiazole, 3-(2-pyridyl)-5-phenyl-1,2,4-oxadiazole, or 3-(2-pyridyl)-5-[3-(trifluoromethyl)phenyl]-1,2,4-oxadiazole; or
b) 3-(2-pyridyl)-5-[3-(trifluoromethoxy)phenyl]-1,2,4-oxadiazole, 3-(2-pyridyl)-5-(2,3-difluorophenyl)-1,2,4-oxadiazole, 3-(2-pyridyl)-5-(3,5-dimethoxyphenyl)-1,2,4-oxadiazole, 3-(5-fluoropyrid-2-yl)-5-(3,5-dimethoxyphenyl)-1,2,4-oxadiazole, 3-(5-methoxypyrid-2-yl)-5-(3-cyanophenyl)-1,2,4-oxadiazole, 3-(2-pyridyl)-5-(3-nitrophenyl)-1,2,4-oxadiazole, 2-[3-chlorophenyl]-4-[pyridin-2-yl]-1,3-oxazole, 2-(3,5-dichlorophenyl)-4-(2-pyridyl)-1,3-oxazole, 2-(3-chlorophenyl)-4-(2-pyridyl)-1,3-oxazole, 2-(3-methoxyphenyl)-4-(2-pyridyl)-1,3-oxazole, 2-(2-chlorophenyl)-4-(2-pyridyl)-1,3-oxazole, 2-(3-trifluorophenyl)-4-(2-pyridyl)-1,3-oxazole, 2-(3-methylphenyl)-4-(2-pyridyl)-1,3-oxazole, 2-(3-trifluoromethoxyphenyl)-4-(2-pyridyl)-1,3-oxazole, 2-(2,3-difluorophenyl)-4-(2-pyridyl)-1,3-oxazole, 2-(2,5-difluorophenyl)-4-(2-pyridyl)-1,3-oxazole, 2-(3,5-difluorophenyl)-4-(2-pyridyl)-1,3-oxazole, 2-(3-cyanophenyl)-4-(2-pyridyl)-1,3-oxazole, 2-(3,5-dimethoxyphenyl)-4-(2-pyridyl)-1,3-oxazole, 2-(2,3-dichlorophenyl)-4-(2-pyridyl)-1,3-oxazole, 2-(3-chloro-5-cyanophenyl)-4-(2-pyridyl)-1,3-oxazole, 2-(3-fluoro-5-cyanophenyl)-4-(2-pyridyl)-1,3-oxazole, 2-(3-chloro-5-fluorophenyl)-4-(2-pyridyl)-1,3-oxazole, 2-(3-cyanophenyl)-4-(5-chloropyrid-2-yl)-1,3-oxazole, 2-(3-cyanophenyl)-4-(5-fluoropyrid-2-yl)-1,3-oxazole, 2-(3-cyano-5-fluorophenyl)-4-(5-fluoropyrid-2-yl)-1,3-oxazole, 2-(3-cyanophenyl)-4-(3-fluoropyrid-2-yl)-1,3-oxazole, 2-(3,5-dimethoxyphenyl)-4-(5-fluoropyrid-2-yl)-1,3-oxazole, 2-(3-cyanophenyl)-4-(5-methoxypyrid-2-yl)-1,3-oxazole, 2-(3-cyanophenyl)-4-(3-chloro-5-trifluoromethylpyrid-2-yl)-1,3-oxazole, 2-(5-chloro-2-methoxyphenyl)-4-(2-pyridyl)-1,3-oxazole, 2-(2-chloro-5-methylthiophenyl)-4-(2-pyridyl)-1,3-oxazole, 2-(2-bromo-5-methoxyphenyl)-4-(2-pyridyl)-1,3-oxazole, 2-(2,5,6-trifluorophenyl)-4-(2-pyridyl)-1,3-oxazole, 2-[3-chlorophenyl]-4-[pyridin-2-yl]-1,3-oxazole, 2-(2,5,6-trifluorophenyl)-4-(2-pyridyl)-1,3-oxazole, 2-(3-nitrophenyl)-4-(2-pyridyl)-1,3-oxazole, 2-(3-bromophenyl)-4-(2-pyridyl)-1,3-oxazole, or a pharmaceutically acceptable salt thereof.

In a preferred embodiment of the invention, the compound is selected from the group consisting of 3-(2-pyridyl)-5-(3,5-dichlorophenyl)-1,2,4-oxadiazole, 3-(2-pyridyl)-5-(3-chlorophenyl)-1,2,4-oxadiazole, 3-(2-pyridyl)-5-(3-methoxyphenyl)-1,2,4-oxadiazole, 3-(2-pyridyl)-5-(2-chlorophenyl)-1,2,4-oxadiazole, 3-(2-pyridyl)-5-[3-(trifluoromethyl)phenyl]-1,2,4-oxadiazole, 3-(2-pyridyl)-5-(3-methylphenyl)-1,2,4-oxadiazole, 3-(2-pyridyl)-5-(1-naphthyl)-1,2,4-oxadiamle, 3-(2-pyridyl)-5-[3-(trifluoromethoxy)phenyl]-1,2,4-oxadiazole, 3-(2-pyridyl)-5-(2,3-difluorophenyl)-1,2,4-oxadiazole, 3-(2-pyridyl)-5-(2,5-difluorophenyl)-1,2,4-oxadiazole, 3-(2-pyridyl)-5-(3,5-difluorophenyl)-1,2,4-oxadiazole, 3-(2-pyridyl)-5-(3-cyanophenyl)-1,2,4-oxadiazole, 3-(2-pyridyl)-5-(3,5-dimethoxyphenyl)-1,2,4-oxadiazol, 3-(2-pyridyl)-5-(2,3-dichlorophenyl)-1,2,4-oxadiazole, 3-(2-pyridyl)-5-(3-chloro-5-cyanophenyl)-1,2,4-oxadiazole, 3-(2-pyridyl)-5-(3-fluoro-5-cyanophenyl)-1,2,4-oxadiazole; 3-(2-pyridyl)-5-(3-chloro-5-fluorophenyl)-1,2,4-oxadiazole, 3-(5-chloropyrid-2-yl)-5-(3-cyanophenyl)-1,2,4-oxadiazole, 3-(5-fluoropyrid-2-yl)-5-(3-cyanophenyl)-1,2,4-oxadiazole, 3-(5-fluoropyrid-2-yl)-5-(3-cyano-5-fluorophenyl)-1,2,4-oxadiazole, 3-(3-fluoropyrid-2-yl)-5-(3-cyanophenyl)-1,2,4-oxadiazole, 3-(5-fluoropyrid-2-yl)-5-(3,5-dimethoxyphenyl)-1,2,4-oxadiazole, 3-(5-methoxypyrid-2-yl)-5-(3-cyanophenyl)-1,2,4-oxadiazole, 3-(2-quinolinyl)-5-(3-cyanophenyl)-1,2,4-oxadiazole, 3-(3-chloro-5-trifluoromethylpyrid-2-yl)-5-(3-cyanophenyl)-1,2,4-oxadiazole, 3-(2-pyridyl)-5-(5-chloro-2-methoxyphenyl)-1,2,4-oxadiazole, 3-(2-pyridyl)-5-(2-chloro-5-methylthiophenyl)-1,2,4-oxadiazole, 3-(2-pyridyl)-5-(2-bromo-5-methoxyphenyl)-1,2,4-oxadiazole, 3-(2-pyridyl)-5-(2,5,6-trifluorophenyl)-1,2,4-oxadiazole, 2-[3-chlorophenyl]-4-[pyridin-2-yl]-1,3-oxazole and 3-(2-pyridyl)-5-(2,5,6-trifluorophenyl)-1,2,4-oxadiazole, 3-(2-pyridyl)-5-(3-nitrophenyl)-1,2,4-oxadiazole, 3-(2-pyridyl)-5-(3-bromophenyl)-1,2,4-oxadiazole and pharmaceutically acceptable salts thereof.

In accordance with another embodiment of the invention, there has been provided a pharmaceutical composition comprising a compound as set forth above, together with a pharmaceutically acceptable diluent or excipient.

In accordance with still another embodiment of the invention, there has been provided a method of making a compound as set forth above. Specifically, compounds of the invention generally can be prepared by formation of the G moiety between two precursor compounds containing suitable Ar¹ and Ar² moieties. When the linker contains an 1,2,4-oxadiazole, the heterocycle may be formed using well known techniques, such as reaction between an amidoxime and an acid chloride, or by the reaction of an amidoxime and an acylimidazole. An illustration of such a transformation is provided in Examples 3 through 6, below.

Amidoximes can be prepared using well known techniques by the reaction of an Ar¹ substituted nitrile with hydroxylamine. An illustration of such a transformation is provided below in Example 1.

In most cases, the precursor Ar² carbonyl chlorides are readily available, or may be prepared using straightforward techniques of organic chemistry. For example, carboxylic acids may be converted into the corresponding acid chlorides by reaction with, for example, thionyl chloride or oxalyl chloride.

In the case where the linker contains a 1,3-oxazole, compounds were prepared from the procedure similar to that given by Kelly *et al*., *J. Org. Chem. 61*, 4623-4633 (1996). 3,5-Disubstituted-1,3-Oxazoles were prepared by reacting a haloketone with carboxamide in refluxing toluene for 3 days. The resulting mixture was allowed to cool to room temperature, the solvent was removed and the residue was purified.

In accordance with a still further embodiment of the invention, there has been provided a use of the compounds defined above for the preparation of a medicament for the treatment of diseases or disorders associated with metabotropic glutamate receptors.

The disease or disorder is preferably selected from the group consisting of as senile dementia, Parkinson's disease, Alzheimer's disease, Huntington's Chorea, pain, migraine headaches, epilepsy, head trauma, anoxic and ischemic injuries, psychiatric disorders such as schizophrenia, depression, anxiety, diabetic retinopathies, glaucoma, tinnitus, diabetic neuropathies, chemotherapy induced neuropathies, post-herpetic neuralgia, and trigeminal neuralgia.

Other objects, features and advantages of the present invention will become apparent from the following detailed description.

### BRIEF DESCRIPTION OF THE DRAWINGS

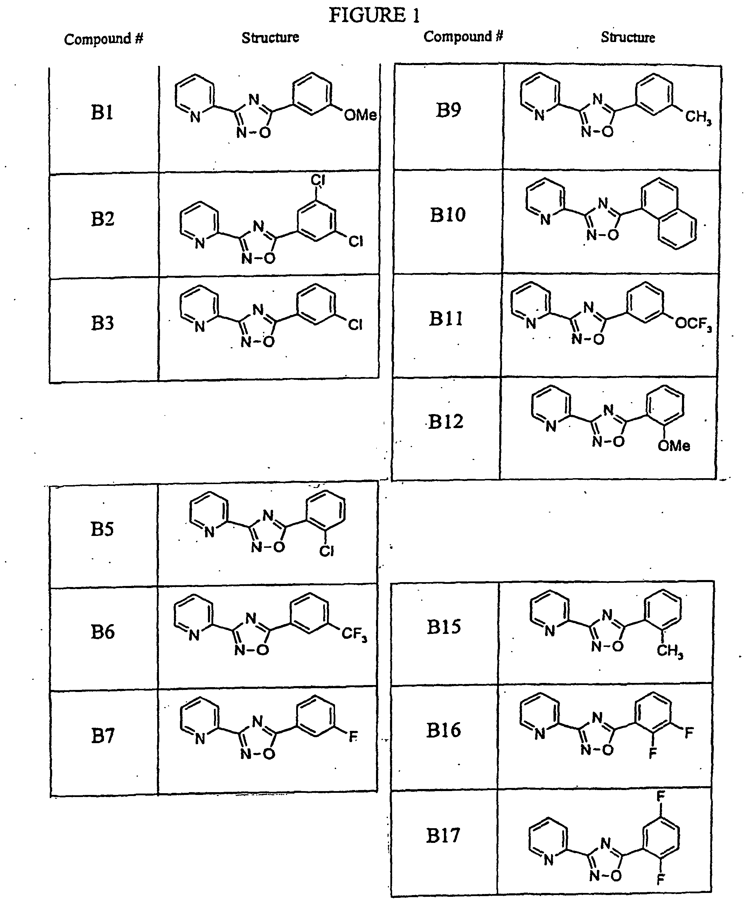

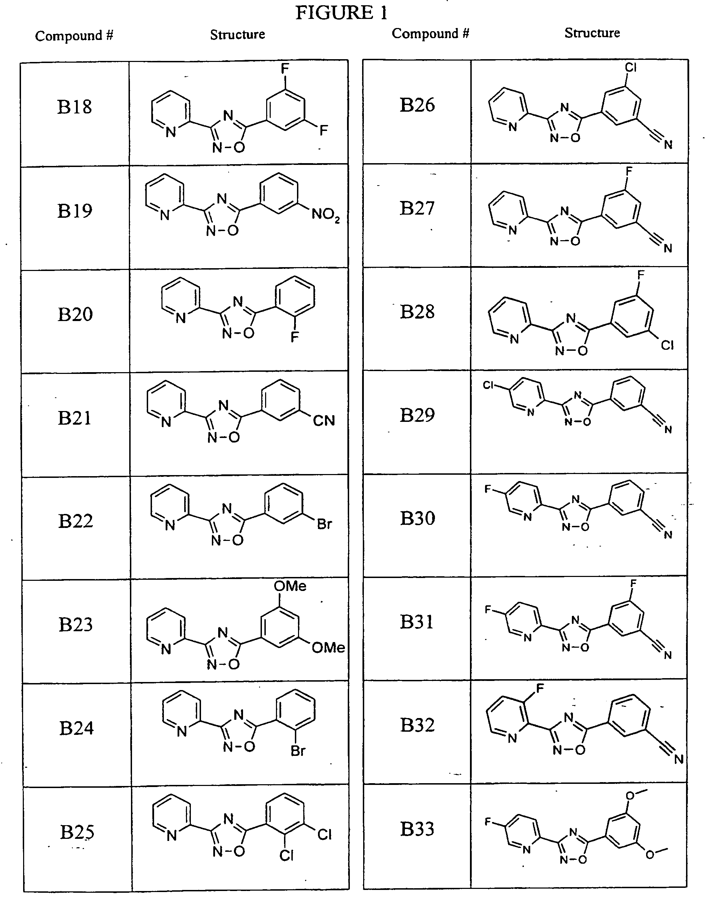

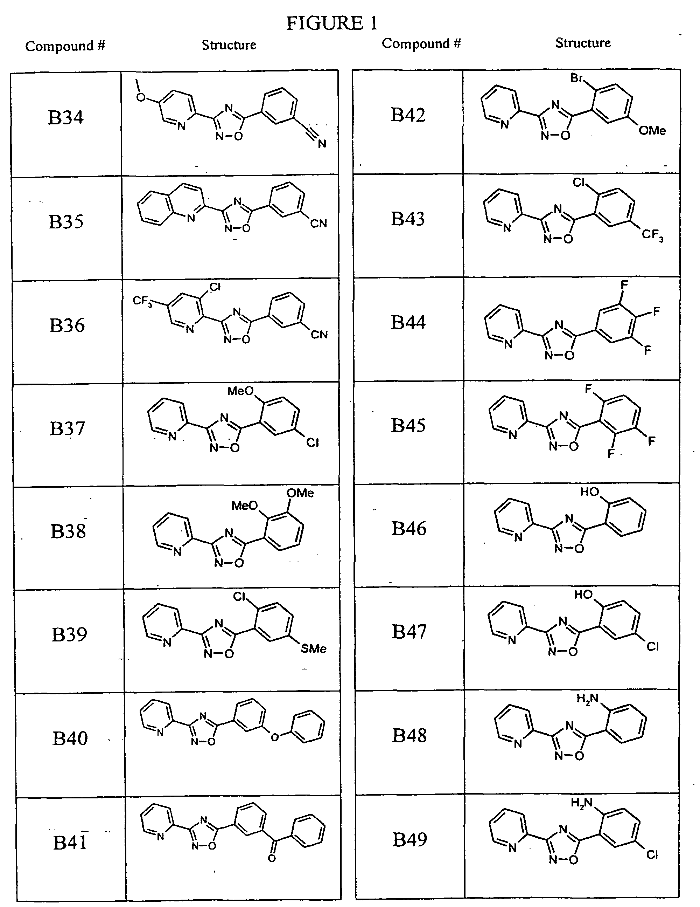

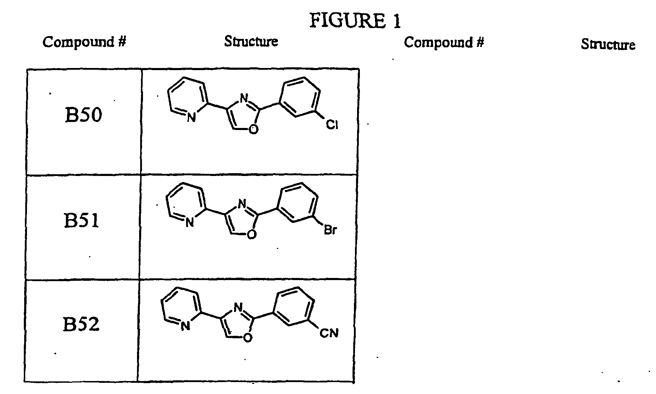

Figure 1 shows illustrative compounds of the invention.

### DETAILED DESCRIPTION OF THE INVENTION

The present invention provides compounds as defined above that are potent and selective antagonists of mGluR5.

### Preparation of mGluR Group I antagonists

Many starting materials for preparing the compounds of the present invention are available from commercial sources, such as Aldrich Chemical Company (Milwaukee, WI). Moreover, compounds of the invention are readily prepared, from available precursors, using straightforward transformations which are well known in the art. The skilled artisan will recognize that mGluR Group I antagonists, according to the invention, can be prepared via methodology that is well known, using widely recognized techniques of organic chemistry. Suitable reactions are described in standard textbooks of organic chemistry. For example, see March, ADVANCED ORGANIC CHEMISTRY, 2d ed., McGraw Hill (1977).

More specifically, compounds of the invention generally can be prepared by formation of the G moiety between two precursor compounds containing suitable Ar¹ and Ar² moieties. When the linker contains a 1,2,4-oxadiazole, the heterocycle may be formed using well known techniques, such as reaction between an amidoxime and an acid chloride, or by the reaction of an amidoxime and an acylimidazole. An illustration of such a transformation is provided in Examples 3 through 6, below.

Amidoximes can be prepared using well known techniques by the reaction of an Ar¹ substituted nitrile with hydroxylamine. An illustration of such a transformation is provided below in Example 1.

In most cases, the precursor Ar² acid chlorides are readily available, or may be prepared using straightforward techniques of organic chemistry. For example, carboxylic acids may be converted into the corresponding acid chlorides by reaction with, for example, thionyl chloride or oxalyl chloride.

In the case where the linker contains a 1,3-oxazole, compounds were prepared using a procedure similar to that given by Kelly *et al*., *J. Org. Chem. 61*, 4623-4633 (1996). Thus, 3,5-Disubstituted-1,3-Oxazoles were prepared by mixing a haloketone with carboxamide in refluxing toluene for 3 days. The resulting mixture was allowed to cool to room temperature, the solvent was removed and the residue was purified.

### Testing of compounds for mGluR Group I antagonist activity

The pharmacological properties of the compounds of the invention can be analyzed using standard assays for functional activity. Examples of glutamate receptor assays are well known in the art, for example, see Aramori *et al*., *Neuron 8*:757 (1992); Tanabe *et* al., *Neuron* 8:169 (1992); Miller *et al., J. Neuroscience* 15: 6103 (1995); Balazs, *et al., J. Neurochemistry* 69:151 (1997).

Conveniently, the compounds of the invention can be studied by means of an assay that measures the mobilization intracellular calcium, [Ca²⁺]ᵢ in cells expressing mGluR5 that can bind the compounds. A well-known cell line which is suitable for this purpose is described in Miller *et al*., *J. Neuroscience* 15: 6103 (1995), the contents of which are hereby incorporated by reference. It has been shown that exposure of rat astrocytes to the growth factors basic fibroblast growth factor, EGF, or transforming growth factor-α markedly increased the protein expression and functional activity of endogenous mGluR5 (Miller *et al., J. Neuroscience, 15(9)*: 6103-6109, 1995).

In brief, primary astrocyte cultures were prepared from 3-5 day old Sprague-Dawley rat pups using a modification of Miller *et al.* and were plated on poly-L lysine coated flasks in Dulbecco's modified Eagle's medium (DMEM) containing fetal calf serum (FCS). For cuvette analysis, cultures were up-regulated with growth factors in flasks for 3-5 days, then harvested and prepared for measurement of [Ca²⁺]ᵢ mobilization as previously described (Nemeth et al., 1998).

For fluorescent imaging plate reader (FLIPR) analysis, cells were seeded on poly-D lysine coated clear bottom 96-well plates with black sides and analysis of [Ca²⁺]ᵢ mobilization was performed 3 days following the growth factor up-regulation.

FLIPR experiments were carried out using a laser setting of 0.800 W and a 0.4 second CCD camera shutter speed. Each FLIPR experiment was initiated with 180 µL of buffer present in each well of the cell plate. After each addition of compound, the fluorescence signal was sampled 50 times at 1 second intervals followed by 3 samples at 5 second intervals. Responses were measured as the peak height of the response within the sample period.

EC₅₀ and IC₅₀ determinations -were made from data obtained from 8 point concentration response curves (CRC) performed in duplicate. Agonist CRC were generated by scaling all responses to the maximal response observed for the plate. Antagonist block of the agonist challenge was normalized to the average response of the agonist challenge in 14 control wells on the same plate. A detailed protocol for testing the compounds of the invention is provided below at Example 4.

### Preparation of pharmaceutical compositions containing mGluR antagonists, and their use in treating neurological disorders

The compounds of the present invention are useful for treating neurological disorders or diseases. While these compounds typically will be used in therapy for human patients, they also can be used in veterinary medicine, to treat similar or identical diseases.

In therapeutic and/or diagnostic applications, the compounds of the invention can be formulated for a variety of modes of administration, including systemic and topical or localized administration. Techniques and formulations generally may be found in REMINGTON'S PHARMACEUTICAL SCIENCES (18th ed.), Mack Publishing Co. (1990).

The compounds according to the invention are effective over a wide dosage range. For example, in the treatment of adult humans, dosages from about 0.01 to about 1000 mg per 60-70 kg adult, preferably from about 0.5 to about 100 mg per 60-70 kg adult, per [day] dose may be used. A more preferable dosage is about 2 mg to about 70 mg per 60-70 kg adult per [day] dose. The exact dosage will depend upon the route of administration, the form in which the compound is administered, the subject to be treated, the body weight of the subject to be treated, and the preference and experience of the attending physician.

Pharmaceutically acceptable salts arc generally well known to those of ordinary skill in the art, and may include, by way of example but not limitation, acetate, benzenesulfonate, besylate, benzoate, bicarbonate, bitartrate, bromide, calcium edetate, camsylate, carbonate, citrate, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, malate, maleate, mandelate, mesylate, mucate, napsylate, nitrate, pamoate (embonate), pantothenate, phosphate/disphosphate, polygalacturonate, salicylate, stearate, subacetate, succinate, sulfate, tannate, tartrate, or teoclate. Other pharmaceutically acceptable salts may be found, for example, in REMINGTON'S PHARMACEUTICAL SCIENCES (18th ed.), *supra.*

Preferred pharmaceutically acceptable salts include, for example, acetate, benzoate, bromide, carbonate, citrate, gluconate, hydrobromide, hydrochloride, maleate, mesylate, napsylate, pamoate (embonate), phosphate, salicylate, succinate, sulfate, or tartrate.

Depending on the specific conditions being treated, such agents may be formulated into liquid or solid dosage forms and administered systemically or locally. The agents may be delivered, for example, in a timed- or sustained-release form as is known to those skilled in the art. Techniques for formulation and administration may be found in REMINGTON'S PHARMACEUTICAL SCIENCES; (18th ed.), *supra*. Suitable routes may include oral, buccal, sublingual, rectal, transdermal, vaginal, transmucosal, nasal or intestinal administration; parenteral delivery, including intramuscular, subcutaneous, intramedullary injections, as well as intrathecal, direct intraventricular, intravenous, intraperitoneal, intranasal, or intraocular injections, *inter alia*.

For injection, the agents of the invention may be formulated in aqueous solutions, preferably in physiologically compatible buffers such as Hank's solution, Ringer's solution, or physiological saline buffer. For transmucosal administration, penetrants appropriate to the barrier to be permeated are used in the formulation. Such penetrants generally are known in the art.

Use of pharmaceutically acceptable carriers to formulate the compounds herein disclosed for the practice of the invention into dosages suitable for systemic administration is within the scope of the invention. With proper choice of carrier and suitable manufacturing practice, the compositions of the present invention, in particular, those formulated as solutions, may be administered parenterally, such as by intravenous injection. The compounds can be formulated readily using pharmaceutically acceptable carriers well known in the art into dosages suitable for oral administration. Such carriers enable the compounds of the invention to be formulated as tablets, pills, capsules, liquids, gels, syrups, slurries, suspensions and the like, for oral ingestion by a patient to be treated.

Pharmaceutical compositions suitable for use in the present invention include compositions wherein the active ingredients are contained in an effective amount to achieve its intended purpose. Determination of the effective amounts is well within the capability of those skilled in the art, especially in light of the detailed disclosure provided herein.

In addition to the active ingredients, these pharmaceutical compositions may contain suitable pharmaceutically acceptable carriers comprising excipients and auxiliaries which facilitate processing of the active compounds into preparations which can be used pharmaceutically. The preparations formulated for oral administration may be in the form of tablets, dragees, capsules, or solutions.

Pharmaceutical preparations for oral use can be obtained by combining the active compounds with solid excipients, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations, for example, maize starch, wheat starch, rice starch, potato starch, gelatin, gum tragacanth, methyl cellulose, hydroxypropylmethyl-cellulose, sodium carboxymethyl-cellulose (CMC), and/or polyvinylpyrrolidone (PVP: povidone). If desired, disintegrating agents may be added, such as the cross-linked polyvinylpyrrolidone, agar, or alginic acid or a salt thereof such as sodium alginate.

Dragee cores are provided with suitable coatings. For this purpose, concentrated sugar solutions may be used, which may optionally contain gum arabic, talc, polyvinylpyrrolidone, carbopol gel, polyethylene glycol (PEG), and/or titanium dioxide, lacquer solutions, and suitable organic solvents or solvent mixtures. Dye-stuffs or pigments may be added to the tablets or dragee coatings for identification or to characterize different combinations of active compound doses.

Pharmaceutical preparations which can be used orally include push-fit capsules made of gelatin, as well as soft, sealed capsules made of gelatin, and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols (PEGs). In addition, stabilizers may be added.

The present invention, thus generally described, will be understood more readily by reference to the following examples, which are provided by way of illustration and are not intended to be limiting of the present invention.

### EXAMPLES

### General Experimental Methods

Capillary gas chromatographic and mass spectral data were obtained using a Hewlett-Packard (HP) 5890 Series II Gas Chromatograph coupled to an HP 5971 Series Mass Selective Detector [Ultra-2 Ultra Performance Capillary Column (crosslinked 5% PhMe silicone); column length, 25 m; column i.d., 0.20 mm; helium flow rate, 60 mL/min; injector temp., 250 °C; temperature program, 20 °C/min from 125 to 325 °C for 10 min, then held constant at 325 °C for 6 min]. Thin-layer chromatography was performed using Analtech Uniplate 250-µm silica gel HF TLC plates. UV light sometimes in conjunction with ninhydrin and Dragendorff's spray reagents (Sigma Chemical Co.) were used for detecting compounds on the TLC plates. Most reagents used in reactions were purchased from the Aldrich Chemical Co. (Milwaukee, WI), Sigma Chemical Co. (Saint Louis, MO), Fluka Chemical Corp. (Milwaukee, WI), Fisher Scientific (Pittsburgh, PA), TCI America (Portland, OR), or Lancaster Synthesis (Windham, NH).

### Example 1: Synthesis of amidoxime intermediates

### Pyrid-2-ylamidoxime

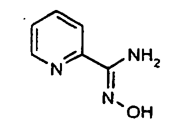

Using the procedure of Shine et al., *J. Heterocyclic Chem.* (1989) 26:125-128, hydroxylamine hydrochloride (7.65 g, 110 mmol) in ethanol (100 mL) was treated with a solution of sodium hydroxide (11 mL of 10 *N,* 110 mmol). A precipitate quickly formed and the reaction mixture was stirred at ambient temperature for 30 min. The inorganic precipitate was filtered and rinsed with ethanol (100 mL). The filtrate and ethanol washings were combined and treated with 2-cyanopyridine (10.4 g, 100 mmol). The reaction mixture was heated at reflux for 20 hours. The volatiles were then removed *in vacuo* to afford 13.3 g (97%) of pyrid-2-ylamidoxime.

### 5-Chloropyrid-2-ylamidoxime

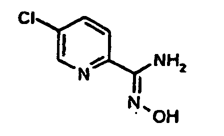

A mixture of 2,5-dichloropyridine (1.48 g, 10 mmol), zinc cyanide (705 mg, 6 mmol), zinc (dust, 29 mg, 0.45 mmol), [1,1'-bis(diphenylphosphino)ferrocene] dichloropalladium(II), complex with dichloromethane (1:1) (0.18 g, 0.22 mmol) in *N,N*-dimethylformamide (10 mL) was heated at reflux for 5 hours. After cooling, the reaction was diluted with ethyl acetate and extracted with water and brine. Silica gel chromatography afforded 735 mg (53 %) of 2-cyano-5-chloropyridine.

Using the general procedure for the synthesis of amidoximes, 2-cyano-5-chloropyridine (735 mg, 5.3 mmol), a solution of hydroxylamine hydrochloride (1.2 mL of 5 M, 6 mmol) in ethanol (7 mL), and sodium hydroxide (0.61 mL of 10 *N*, 6.1 mmol), were heated at reflux for 24 hours. Standard work up afforded 707 mg (77%) of 5-chloropyrid-2-ylamidoxime.

### 5-Fluoropyrid-2-ylamidoxime

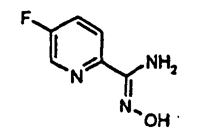

A mixture of 2-cyano-5-chloropyridine (1 g, 7.22 mmol) and potassium fluoride (1.26 g, 21.68 mmol) in 1-methyl-2-pyrrolidinone (25 mL) was heated at reflux 18 hours. After cooling, the reaction was diluted with ethyl acetate and extracted with water and brine. The organic solvents were then removed *in vacuo*. Silica gel chromatography of the residue afforded 425 mg (48 %) of 2-cyano-5-fluoropyridine.

Using the general procedure for the synthesis of amidoximes, 2-cyano-5-fluoropyridine (425 mg, 3.48 mmol), a solution of hydroxylamine hydrochloride (0.79 ml of 5 M, 3.95 mmol) in ethanol (5 mL), and sodium hydroxide (0.398 mL of 10 N, 3.98 mmol) were heated at reflux for 24 hours. Standard work up afforded 330 mg (61 %) of 5-fluoropyrid-2-ylamidoxime.

### 5-Methoxypyrid-2-ylamidoxime

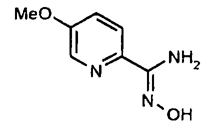

A solution of 2-cyano-5-fluoropyridine (0.65 g, 5.3 mmol) in sodium methoxide (1.83 mL of 25% wt. solution in methanol, 7.95 mmol) was stirred at 0 °C for 1.5 hours and 2 hours at ambient temperature. The reaction was then diluted with ethyl acetate and washed with water and brine. Removal of the solvent *in vacuo* afforded 304 mg (43%) of 2-cyano-5-methoxypyridine.

Using the general procedure for the synthesis of amidoximes, 2-cyano-5-methoxypyridine (270 mg, 2.01 mmol), a solution of hydroxylamine hydrochloride (0.457 ml of 5 M, 2.28 mmol) in ethanol (4 mL), and sodium hydroxide (0.230 mL of 10 *N*, 2.30 mmol) were heated at reflux for 24 hours. Standard work up afforded 79 mg (24%) of 5-methoxypyrid-2-ylamidoxime.

### 3-Fluoropyrid-2-ylamidoxime

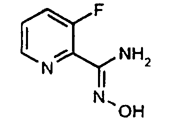

A mixture of 2,3-dichloropyridine (1.48 g, 10 mmol), zinc cyanide (705 mg, 6 mmol,), zinc (dust, 29 mg, 0.45 mmol), [1,1'-bis(diphenylphosphino)ferrocene] dichloropalladium(II), complex with dichloromethane (1:1) (0.18 g, 0.22 mol) in *N,N*-dimethylformamide (10 mL) was heated at reflux for 5 hours. After cooling, the reaction was diluted with ethyl acetate and extracted with water and brine. Removal of the solvent and silica gel chromatography afforded 1.05 g (76%) of 2-cyano-3-chloropyridine.

A solution of 2-cyano-3-chloropyridine (1 g, 7.22 mmol) in 1-methyl-2-pyrrolidinone (25 mL) was treated with potassium fluoride (1.26 g, 21.68 mmol) and heated at reflux for 18 hours. After cooling, the reaction was diluted with ethyl acetate and extracted with water and brine. Silica gel chromatography afforded 442 mg (50%) of 2-cyano-3-fluoropyridine.

Using the general procedure for the synthesis of amidoximes, 2-cyano-3-fluoropyridine (442 mg, 3.62 mmol), a solution of hydroxylamine hydrochloride (0.82 mL of 5 M, 4.1 mmol) in ethanol (5 mL), and sodium hydroxide (0.415 ml of 10 N, 4.15 mmol) were heated at reflux for 24 hours. Standard work up afforded 368 mg (66%) of 3-fluoropyrid-2-ylamidoxime.

### Quinol-2-ylamidoxime

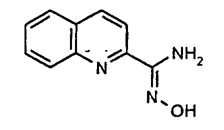

Using the general procedure for the synthesis of amidoximes, 2-quinolinecarbonitrile (1.02 g, 6.6 mmol), a solution of hydroxylamine hydrochloride (1.44 mL of 5 N solution, 7.2 mmol) in ethanol (10 mL), and sodium hydroxide (0.72 mL of 10 N solution, 7.2 mmol) were heated at reflux for 18 hours. Standard work up afforded 990 mg (80%) of quinol-2-ylamidoxime.

### Example 2: Synthesis of carboxylic acid intermediates

### 3-Chloro-5-cyanobenzoic acid

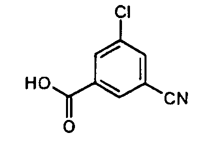

A mixture of methyl 3,5-dichlorobenzoate (14.66 g, 71.5 mmol), zinc cyanide (5.04 g, 42.9 mmol) zinc (dust, 0.21 g, 3.21mmol), [1,1'Bis(diphenylphosphino)ferrocene] dichloropalladium(II), complex with dichloromethane (1:1) (1.3 g, 1.57 mmol) in *N,N*-dimethylformamide (70 mL) was heated at reflux for 5 hours. After cooling the reaction was diluted with ethyl acetate and extracted with water and brine. Silica gel chromatography afforded 2.34g (17%) methyl 2-chloro-5-cyanobenzoate.

The intermediate ester was treated with a solution of sodium hydroxide (7.5 mL of 4 N solution, 30 mmol) in methanol (50 mL) and stirred at ambient temperature for 18 hours. The solvent was removed *in vacuo* and the residue dissolved in ethyl acetate. The organic solution was washed with 5% HCl and brine. Removal of the solvent afforded 1.8 g (83%) of 3-chloro-5-cyanobenzoic acid.

### 3-Chloro-5-fluorobenzoic acid

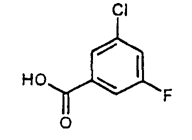

A mixture of 1-bromo-3-chloro-5-fluorobenzene (25.0 g, 120 mmol), zinc cyanide (8.45 g, 72 mmol) zinc (dust, 235 mg, 3.6 mmol), [1,1'Bis(diphenylphosphino)ferrocene] dichloropalladium(II), complex with dichloromethane (1:1) (1.5 g, 1.8 mmol) in *N,N*-dimethylformamide (70 ml) was heated at reflux for 1 hour. After cooling the reaction was diluted with ethyl acetate and extracted with water and brine. Silica gel chromatography afforded 15.9g (85 %) 3-chloro-5-fluorobenzonitrile.

The intermediate nitrile was treated with a solution of sodium hydroxide (100 mL of 10 N solution, 1 mol) in 100 mL water and heated at reflux for 2 hours. After this time the solution was cooled and acidified with concentrated hydrochloric acid. Extraction with dichloromethane and evaporation of the solvent, afforded 15.14g (85%) of 3-chloro-5-fluorobenzoic acid.

### 3-Fluoro-5-cyanobenzoic acid

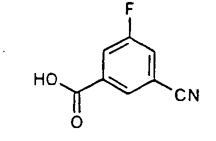

3-Chloro-5-fluorobenzoic acid (13.74g, 78.7 mmol) was treated with 50 ml thionyl chloride and heated at reflux for 2 hours. The excess thionyl chloride was removed *in vacuo* and the residue treated with 100 ml dry methanol to afford 13.6g (92%) of methyl 3-chloro-5-fluorobenzoate.

A mixture of the methyl 3-chloro-5-fluorobenzoate, zinc cyanide (8.46g, 72.3 mmol) zinc (dust, 235 mg, 3.6mmol), [1,1'bis(diphenylphosphino)ferrocene] dichloropalladium(II), complex with dichloromethane (1:1) (1.5 g, 1.8 mmol) in *N,N*-dimethylformamide (70 ml) was heated at reflux for 1 hour. The reaction was cooled to ambient temperature and diluted with ethyl acetate. The organic solution was extracted with water and brine and concentrated *in vacuo*, to afford crude methyl 3-chloro-5-cyanobenzoate.

The crude methyl 3-chloro-5-cyanobenzoate was treated with a solution of sodium hydroxide (45 ml of 4 *N* solution, 180 mmol) in methanol (350 mL) at ambient temperature for 4 hours. The solvent was *removed in vacuo* and the residue dissolved in ethyl acetate. The organic solution was washed with 5% aqueous HCl and brine. Silica gel chromatography afforded 7.0 g (54%) of 3-fluoro-5-cyanobenzoic acid.

### Example 3: Synthesis of 3,5-disubstituted-1,2,4-oxadiazoles from acid chlorides

In general, modifications were made from the procedures given Shine et al., *J. Heterocyclic Chem.* (1989) *26*:125-128. 3,5-Disubstituted-1,2,4-oxadiazoles were typically made by adding an acylchloride to a solution of an amidoxime in pyridine after which the reaction mixture was either heated to reflux or placed in a sealed tube and heated. Typically, the oxadiazoles were isolated by precipitating with cold water and filtering or by extraction with an organic solvent. If necessary, the oxadiazoles were purified by chromatography or recrystallization.

### 3-(2-Pyridyl)-5-(3,5-dichlorophenyl)-1,2,4-oxadiazole (NPS 64982) (404) B2

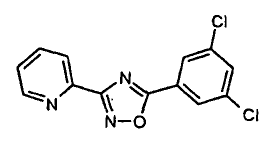

A mixture of 3,5-dichlorobenzoyl chloride (2.1 g, 10 mmol) and pyrid-2-ylamidoxime (1.37 g, 10 mmol) in pyridine (5 mL) was heated in sealed tube at 190 °C for 2 hours. After this time, the reaction mixture was added to ice cold water to precipitate the oxadiazole. The solid was collected by filtration, washed with water and then recrystallized from ethanol to yield 2.1 g (72%) of 3-(2-pyridyl)-5-(3,5-dichlorophenyl)-1,2,4-oxadiazole: mp 162-166 °C; GC/EI-MS gave *m*/*z* (rel. int.) 291 (M⁺, 38), 293 (25), 261 (1), 173 (6), 145 (13), 120 (100), 90 (20), 78 (28), 51 (15).

### 3-(2-Pyridyl)-5-(3-chlorophenyl)-1,2,4-oxadiazole (NPS 64983) (405) B3

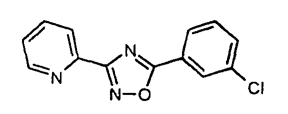

Using the general procedure for the synthesis of 1,2,4-oxadiazoles, 3-chlorobenzoyl chloride (127 µL, 1 mmol) and pyrid-2-ylamidoxime (137 mg, 1 mmol) in pyridine (1 mL) were heated at reflux for 4 hours. Standard work up afforded 156 mg (61 %) of 3-(2-pyridyl)-5-(3-chlorophenyl)-1,2,4-oxadiazole: mp 136-140 °C; GC/EI-MS gave *m*/*z* (rel. int.) 257 (M⁺, 64), 259 (21), 227 (3), 120 (100), 111 (22), 90 (24), 78 (32), 75 (26), 51 (20).

### 3-(2-Pyridyl)-5-(3-methoxyphenyl)-1,2,4-oxadiazole (B1)

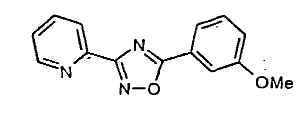

Using the general procedure for the synthesis of 1,2,4-oxadiazoles, 3-anisoyl chloride (151 µL, 1 mmol) and pyrid-2-ylamidoxime (137 mg, 1 mmol) in pyridine (1 mL) were heated at reflux for 4 hours. Standard work up afforded 200 mg (79%) of 3-(2-pyridyl)-5-(3-methoxyphenyl)-1,2,4-oxadiazole: mp 96-99 °C; GC/EI-MS gave *m*/*z* (rel. int.) 253 (M⁺, 100), 223 (3), 179 (3), 135 (74), 133 (90), 92 (27), 78 (29), 77 (32), 64 (23), 63 (23).

### 3-(2-Pyridyl)-5-(2-chlorophenyl)-1,2,4-oxadiazole (B5)

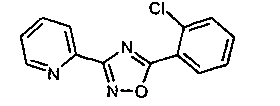

Using the general procedure for the synthesis of 1,2,4-oxadiazoles, 2-chlorobenzoyl chloride (127 µL, 1 mmol) and pyrid-2-ylamidoxime (137 mg, 1 mmol) in pyridine (1 mL) were heated at reflux for 4 hours. Standard work up afforded 157 mg (61%) of 3-(2-pyridyl)-5-(2-chlorophenyl)-1,2,4-oxadiazole: mp 93-94 °C; GC/EI-MS gave *m*/*z* (rel. int.) 257 (M⁺, 76), 259 (26), 227 (4), 139 (11), 120 (100), 111 (21), 90 (27), 78 (35), 75 (29), 51 (21).

### 3-(2-Pyridyl)-5-[3-(trifluoromethyl)phenyl]-1,2,4-oxadiazole (B6)

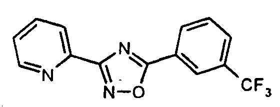

Using the general procedure for the synthesis of 1,2,4-oxadiazoles, 3-(trifluoromethyl)benzoyl chloride (151 µL, 1 mmol) and pyrid-2-ylamidoxime (137 mg, 1 mmol) in pyridine (1 mL) were heated at reflux for 16 hours. Standard work up afforded 233 mg (80%) of 3-(2-pyridyl)-5-[3-(trifluoromethyl)phenyl]-1,2,4-oxadiazole: mp 116-118 °C; GC/EI-MS gave *m*/*z* (rel. int.) 291 (M⁺, 81), 272 (7), 173 (6), 145 (25), 120 (100), 90 (20), 78 (23), 51 (11).

### 3-(2-Pyridyl)-5-(3-fluorophenyl)-1,2,4-oxadiazole (B7)

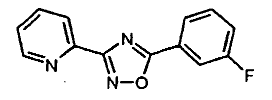

Using the general procedure for the synthesis of 1,2,4-oxadiazoles, 3-fluorobenzoyl chloride (122 µL, 1 mmol) and pyrid-2-ylamidoxime (137 mg, 1 mmol) in pyridine (1 mL) were heated at reflux for 16 hours. Standard work up afforded 176 mg (73%) of 3-(2-pyridyl)-5-(3-fluorophenyl)-1,2,4-oxadiazole: mp 88-98 °C; GC/EI-MS gave *m*/*z* (rel. int.) 241 (M⁺, 95), 211 (5), 120 (100), 107 (13), 95 (30), 90 (21), 78 (27), 75 (19), 51 (15).

### 3-(2-Pyridyl)-5-(3-methylphenyl)-1,2,4-oxadiazole (B9)

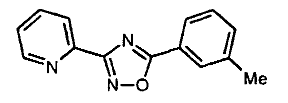

Using the general procedure for the synthesis of 1,2,4-oxadiazoles, 3-toluoyl chloride (264 µL, 2 mmol) and pyrid-2-ylamidoxime (274 mg, 2 mmol) in pyridine (1 mL) were heated in a sealed tube at 200 °C for 2 hours. Standard work up afforded 387 mg (82%) of 3-(2-pyridyl)-5-(3-toluoyl)-1,2,4-oxadiazole: mp 127-128 °C; GC/EI-MS gave *m*/*z* (rel. int.) 237 (M⁺, 100), 222 (2), 207 (8), 120 (68), 117 (24), 91 (29), 90 (29), 78 (32), 65 (26), 51 (23).

### 3-(2-Pyridyl)-5-(1-naphthyl)-1,2,4-oxadiazole (B10)

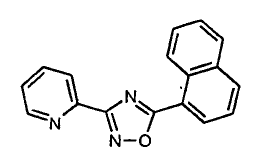

Using the general procedure for the synthesis of 1,2,4-oxadiazoles, 1-naphthoyl chloride (150 µL, 1 mmol) and pyrid-2-ylamidoxime (137 mg, 1 mmol) in pyridine (1 mL) were heated in a sealed tube at 200 °C for 3 hours. Standard work up afforded 50 mg (18%) of 3-(2-pyridyl)-5-(1-naphthyl)-1,2,4-oxadiazole: mp 132-136 °C; GC/EI-MS gave *m*/*z* (rel. int.) 273 (M⁺, 75), 195 (5), 169 (88), 153 (100), 139 (12), 127 (66), 126 (29), 105 (23), 78 (14), 51 (14).

### 3-(2-Pyridyl)-5-[3-(trifluoromethoxy)phenyl]-1,2,4-oxadiazole (B11)

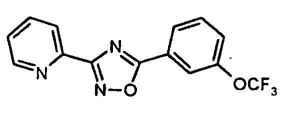

Using the general procedure for the synthesis of 1,2,4-oxadiazoles, 3-(trifluoromethoxy)benzoyl chloride (220 mg, 1 mmol), and pyrid-2-ylamidoxime (137 mg, 1 mmol) in pyridine (1 mL) were heated in a sealed tube at 200 °C for 3 hours. Standard work up afforded 175 mg (57%) of 3-(2-pyridyl)-5-[3-(trifluoromethoxy)phenyl]-1,2,4-oxadiazole: mp 86-88 °C; GC/EI-MS gave *m*/*z* (rel. int.) 307 (M⁺, 73), 277 (3), 222 (3), 189 (6), 161 (5), 120 (100), 78 (21), 69 (17), 51 (10).

### 3-(2-Pyridyl)-5-(2,3-difluorophenyl)-1,2,4-oxadiazole (B16)

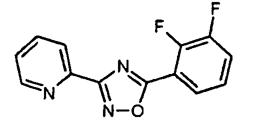

Using the general procedure for the synthesis of 1,2,4-oxadiazoles, 2,3-difluorobenzoyl chloride (124 µL, 1 mmol) and pyrid-2-ylamidoxime (137 mg, 1 mmol) in pyridine (1 mL) were heated at 100 °C for 16 hours. Standard work up afforded 158 mg (61%) of 3-(2-pyridyl)-5-(2,3-difluorophenyl)-1,2,4-oxadiazole: mp 120-121 °C; GC/EI-MS gave *m*/*z* (rel. int) 259 (M⁺, 97), 229 (5), 228 (4), 141 (11), 120 (100), 113 (26), 90 (27), 78 (34), 51 (17).

### 3-(2-Pyridyl)-5-(2,5-difluorophenyl)-1,2,4-oxadiazole (B17)

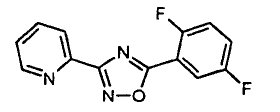

Using the general procedure for the synthesis of 1,2,4-oxadiazoles, 2,5-difluorobenzoyl chloride (124 µL, 1 mmol) and pyrid-2-ylamidoxime (137 mg, 1 mmol) in pyridine (1 mL) were heated at 100 °C for 16 hours. Standard work up afforded 3-(2-pyridyl)-5-(2,5-difluorophenyl)-1,2,4-oxadiazole: mp 120-126 °C; GC/EI-MS gave *m*/*z* (rel. int) 259 (M⁺, 91), 229 (5), 228 (4), 141 (13), 120 (100), 113 (25), 90 (23), 78 (27), 51 (14).

### 3-(2-Pyridyl)-5-(3,5-difluorophenyl)-1,2,4-oxadiazole (B18)

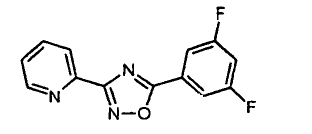

Using the general procedure for the synthesis of 1,2,4-oxadiazoles, 3,5-difluorobenzoyl chloride (1.25 mL, 10 mmol) and pyrid-2-ylamidoxime (1.37 g, 10 mmol) in pyridine (5 mL) were heated in a sealed tube at 200 °C for 4 hours. Standard work up afforded 1.2 g (46%) of 3-(2-pyridyl)-5-(3,5-difluorophenyl)-1,2,4-oxadiazole: mp 115-119 °C; GC/EI-MS gave *m*/*z* (rel. int) 259 (M⁺, 100), 229 (4), 228 (5), 141 (9), 125 (13), 113 (30), 90 (19), 78 (27), 63 (23), 51 (15).

### 3-(2-Pyridyl)-5-(3-cyanophenyl)-1,2,4-oxadiazole (B21)

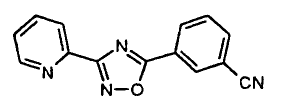

Using the general procedure for the synthesis of 1,2,4-oxadiazoles, 3-cyanobenzoyl chloride (165 mg, 1 mmol) and pyrid-2-ylamidoxime (137 mg, 1 mmol) in pyridine (1 mL) were heated at 100 °C for 72 hours. Standard work up afforded 158 mg (64%) of 3-(2-pyridyl)-5-(3-cyanophenyl)-1,2,4-oxadiazole: mp 148-149 °C; GC/EI-MS gave *m*/*z* (rel. int.) 248 (M⁺, 85), 218 (5), 130 (6), 120 (100), 114 (9), 102 (28), 90 (26), 78 (37), 75 (19), 51 (30).

### 3-(2-Pyridyl)-5-(3,5-dimethoxyphenyl)-1,2,4-oxadiazole (B23)

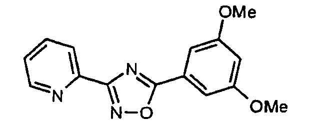

Using the general procedure for the synthesis of 1,2,4-oxadiazoles, 3,5-dimethoxybenzoyl chloride (200 mg, 1 mmol) and pyrid-2-ylamidoxime (137 mg, 1 mmol) in pyridine (1 mL) were heated at 100 °C for 72 hours. Standard work up afforded 210 mg (74%) of 3-(2-pyridyl)-5-(3,5-dimethoxyphenyl)-1,2,4-oxadiazole: mp 145-148 °C; GC/EI-MS gave *m*/*z* (rel. int.) 283 (M⁺, 100), 253 (3), 165 (69), 163 (19), 137 (36), 122 (33), 107 (17), 90 (10), 78 (25), 63 (19), 51 (19).

### 3-(2-Pyridyl)-5-(2,3-dichlorophenyl)-1,2,4-oxadiazole (B25)

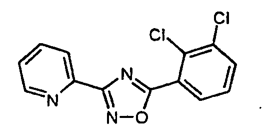

Using the general procedure for the synthesis of 1,2,4-oxadiazoles, 2,3-dichlorobenzoyl chloride (209 mg, 1 mmol) and pyrid-2-ylamidoxime (137 mg, 1 mmol) in pyridine (1 mL) were heated at 100 °C for 48 hours. Standard work up afforded 236 mg (81 %) of 3-(2-pyridyl)-5-(2,3-dichlorophenyl)-1,2,4-oxadiazole: mp 128-133 °C; GC/EI-MS gave *m*/*z* (rel. int.) 291 (M⁺, 66), 293 (43), 256 (6), 173 (10), 145 (11), 120 (100), 90 (19), 78 (27), 51 (14).

### 3-(2-Pyridyl)-5-(3-chloro-5-cyanophenyl)-1,2,4-oxadiazole (B26)

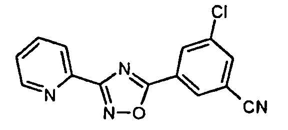

3-Chloro-5-cyanobenzoic acid (0.82 g, 4.97 mmol) was treated with a solution of oxalyl chloride (10 mL of 2.5 M in dichloromethane, 25 mmol) and a catalytic amount of *N,N*-dimethylformamide. The reaction was stirred at ambient temperature for 2.5 hours. The excess oxalyl chloride was removed *in vacuo* to afford 3-chloro-5-cyanobenzoyl chloride.

Using the general procedure for the synthesis of 1,2,4-oxadiazoles, the 3-chloro-5-cyanobenzoyl chloride and pyrid-2-ylamidoxime (682 mg, 5 mmol, 1 equivalent) in pyridine (5 mL) were heated in a sealed tube at 175 °C for 4 hours. Standard work up and recrystallization from 2-propanol afforded 250 mg (19%) of 3-(2-pyridyl)-5-(3-chloro-5-cyanophenyl)-1,2,4-oxadiazole: GC/EI-MS gave *m*/*z* (rel. int.) 282 (M⁺, 100), 283 (18), 284 (34), 251 (4), 136 (10), 120 (53), 100 (10), 78 (15), 51 (6).

### 3-(2-Pyridyl)-5-(3-fluoro-5-cyanophenyl)-1,2,4-oxadiazole (B27)

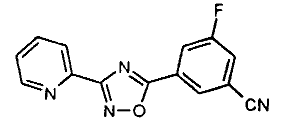

3-Fluoro-5-cyanobenzoic acid (2.5 g, 15.14 mmol) was treated with a solution of oxalyl chloride (30 mL of 2.5 M in dichloromethane, 75 mmol) and a catalytic amount of *N,N*-dimethylformamide. The reaction was stirred at ambient temperature for 2.5 hours. The excess oxalyl chloride was removed *in vacuo* to afford 3-fluoro-5-cyanobenzoyl chloride.

Using the general procedure for the synthesis of 1,2,4-oxadiazoles, the 3-fluoro-5-cyanobenzoyl chloride and pyrid-2-ylamidoxime (2.076 g, 15.15 mmol, 1 equivalent) in pyridine (5 mL) were heated in a sealed tube at 175 °C for 4 hours. Standard work up and recrystallization from 2-propanol afforded 1.5 g (37%) of 3-(2-pyridyl)-5-(3-fluoro-5-cyanophenyl)-1,2,4-oxadiazole: GC/EI-MS gave *m*/*z* (rel. int.) 266 (M⁺, 81), 267 (13), 235 (5), 132 (12), 120 (100), 100 (18), 90 (18), 78 (35), 51 (20).

### 3-(2-Pyridyl)-5-(3-chloro-5-fluorophenyl)-1,2,4-oxadiazole (B28)

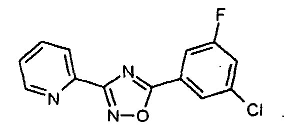

3-Chloro-5-fluorobenzoic acid (400 mg, 2.3 mmol) was treated with a solution of oxalyl chloride (4.6 mL of 2.5 M in dichloromethane, 11.5 mmol) and a catalytic amount of *N,N*-dimethylformamide. The reaction was stirred at ambient temperature for 2.5 hours. The excess oxalyl chloride was removed *in vacuo* to afford 3-chloro-5-fluorobenzoyl chloride.

Using the general procedure for the synthesis of 1,2,4-oxadiazoles, the 3-chloro-5-fluorobenzoyl chloride and pyrid-2-ylamidoxime (314 mg, 2.3 mmol, 1 equivalent) in pyridine (5 mL) were heated in a sealed tube at 175 °C for 4 hours. Standard work up and recrystallization from 2-propanol afforded 250 mg (39%) of 3-(2-pyridyl)-5-(3-chloro-5-fluorophenyl)-1,2,4-oxadiazole: GC/EI-MS gave *m*/*z* (rel. int.) 275(M⁺, 89), 276 (14), 277 (29), 129 (26), 120 (100), 109 (7), 90 (20), 78 (31), 51 (14).

### 3-(5-Chloropyrid-2-yl)-5-(3-cyanophenyl)-1,2,4-oxadiazole (B29)

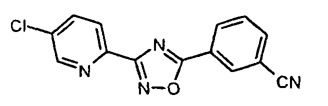

Using the general procedure for the synthesis of 1,2,4-oxadiazoles, 3-cyanobenzoyl chloride (675 mg, 4mmol) and 5-chloropyrid-2-ylamidoxime (686 mg, 4 mmol) in pyridine (5 mL) were heated in a sealed tube at 175 °C for 4 hours. Standard work up and recrystallization from 2-propanol afforded 357 mg (32%) of 3-(5-chloropyrid-2-yl)-5-(3-cyanophenyl)-1,2,4-oxadiazole: GC/EI-MS gave *m*/*z* (rel. int.) 282 (M⁺, 85), 283 (14), 284 (27), 156 (31), 154 (100), 112 (19), 102 (30), 76 (28), 64 (13).

### 3-(5-Fluoropyrid-2-yl)-5-(3-cyanophenyl)-1,2,4-oxadiazole (B30)

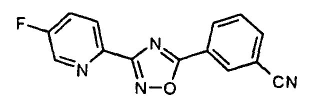

Using the general procedure for the synthesis of 1,2,4-oxadiazoles, 3-cyanobenzoyl chloride (0.534 g, 3.2 mmol) and 5-fluoropyrid-2-ylamidoxime (0.5 g, 3.2 mmol) in pyridine (5 mL) were heated in a sealed tube at 175 °C for 4 hours. Standard work up and recrystallization from 2-propanol afforded 370 mg (43%) of 3-(5-fluoropyrid-2-yl)-5-(3-cyanophenyl)-1,2,4-oxadiazole: GC/EI-MS gave *m*/*z* (rel. int.) 266 (M⁺, 100), 267 (10), 138 (80), 114 (8), 102 (19). 96 (22), 76 (17), 57 (8).

### 3-(5-Fluoropyrid-2-yl)-5-(3-cyano-5-fluorophenyl)-1,2,4-oxadiazole (B31)

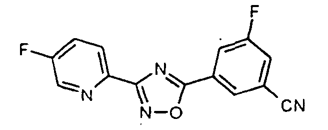

3-Fluoro-5-cyanobenzoic acid (1.0 g, 6 mmol) was treated with a solution of oxalyl chloride (12 mL of 2.5 M in dichloromethane, 30 mmol) and a catalytic amount of *N,N*-dimethylformamide. The reaction was stirred at ambient temperature for 2.5 hours. The excess oxalyl chloride was removed *in vacuo* to afford 3-fluoro-5-cyanbenzoyl chloride.

Using the general procedure for the synthesis of 1,2,4-oxadiazoles, the 3-fluoro-5-cyanbenzoyl chloride (1.1 g, 6 mmol) and 5-fluoropyrid-2-ylamidoxime (0.93 g, 6 mmol) in pyridine (5 mL) were heated in a sealed tube at 175 °C for 4 hours. Standard work up and recrystallization from 2-propanol afforded 0.41 g (24%) of 3-(5-fluoropyrid-2-yl)-5-(3-cyano-5-fluorophenyl)-1,2,4-oxadiazole: GC/EI-MS gave *m*/*z* (rel. int.) 284 (M⁺, 100), 285 (16), 253 (2), 138 (99), 120 (23), 108 (16), 96 (25), 82 (15), 57 (11).

### 3-(3-Fluoropyrid-2-yl)-5-(3-cyanophenyl)-1,2,4-oxadiazole (B32)

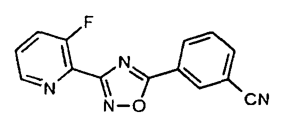

Using the general procedure for the synthesis of 1,2,4-oxadiazoles, 3-cyanobenzoyl chloride (107 mg, 0.64 mmol) and 3-fluoropyrid-2-ylamidoxime (0.1 g, 0.64 mmol) in pyridine (5 mL) were heated in a sealed tube at 175 °C for 4 hours. Standard work up, silica gel chromatography, and recrystallization from 2-propanol, afforded 32 mg (19%) of 3-(3-fluoropyrid-2-yl)-5-(3-cyanophenyl)-1,2,4-oxadiazole: GC/EI-MS gave *m*/*z* (rel. int.) 266 (M⁺, 75), 267 (12), 138(100), 114(11), 102(19), 96 (17), 76 (16), 57 (5), 51 (5).

### 3-(5-Fluoropyrid-2-yl)-5-(3,5-dimethoxyphenyl)-1,2,4-oxadiazole (B33)

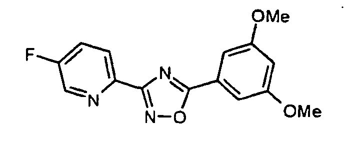

Using the general procedure for the synthesis of 1,2,4-oxadiazoles, 3,5-dimethoxybenzoyl chloride (0.10 g, 0.5 mmol) and 5-fluoropyrid-2-ylamidoxime (78 mg, 0.5 mmol) in pyridine (3 mL) were heated in a sealed tube at 175 °C for 4 hours. Standard work up, silica gel chromatography, and recrystallization from 2-propanol afforded 94 mg (62%) of 3-(5-fluoropyrid-2-yl)-5-(3,5-dimethoxyphenyl)-1,2,4-oxadiazole: GC/EI-MS gave *m*/*z* (rel. int.) 301 (M⁺, 100), 302 (17), 165 (41), 137 (23), 122 (27), 96 (15), 77 (11), 63 (12).

### 3-(5-Methoxypyrid-2-yl)-5-(3-cyanophenyl)-1,2,4-oxadiazole (B34)

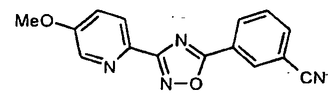

Using the general procedure for the synthesis of 1,2,4-oxadiazoles, 3-cyanobenzoyl chloride (79 mg, 0.47 mmol) and 5-methoxypyrid-2-ylamidoxime (79 mg, 0.47 mmol) in pyridine (2.5 mL) were heated in a sealed tube at 175 °C for 4 hours. Standard work up, silica gel chromatography, and recrystallization from 2-propanol afforded 59 mg (45%) of 3-(5-methoxypyrid-2-yl)-5-(3-cyanophenyl)-1,2,4-oxadiazole: GC/EI-MS gave *m*/*z* (rel. int.) 278 (M⁺, 100), 279 (16), 150 (56), 128 (7), 107 (21), 102 (17), 80 (12), 64 (5).

### 3-(2-Quinolinyl)-5-(3-cyanophenyl)-1,2,4-oxadiazole (B35)

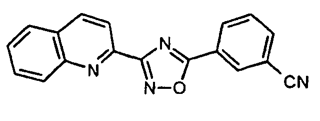

Using the general procedure for the synthesis of 1,2,4-oxadiazoles, 3-cyanobenzoyl chloride (68 mg, 0.41 mmol) and quinol-2-ylamidoxime (75.9 mg, 0.405 mmol) in pyridine (0.5 mL) were heated in a sealed tube at 165 °C for 22 hours. Standard work up, recrystallization from ethanol, and solid phase extraction (SPE) afforded 23.7 mg (20%) of 3-(2-quinolinyl)-5-(3-cyanophenyl)-1,2,4-oxadiazole. ¹H-NMR (CDCl₃), δ (ppm): 8.62 (s, 1H), 8.54 (d, 1H), 8.36 (d, 2H), 8.28 (d, 1H), 7.90 (d, 2H), 7.80 (t, 1H), 7.72 (t, 1H), 7.64 (t, 1H).

### 3-(3-chloro-5-trifluoromethylpyrid-2-yl)-5-(3-cyanophenyl)-1,2,4-oxadiazole (B36)

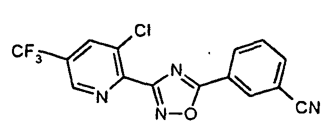

Using the general procedure for the synthesis of 1,2,4-oxadiazoles, 3-cyanobenzoyl chloride (66 mg, 0.40 mmol) and 3-chloro-5-trifluoromethylpyrid-2-ylamidoxime (96.5 mg, 0.403 mmol) in pyridine (0.5 mL) were heated in a sealed tube at 165 °C for 22 hours. Standard work up and solid phase extraction (SPE) afforded 45.9 mg (33%) of 3-(3-chloro-5-trifluoromethylpyrid-2-yl)-5-(3-cyanophenyl)-1,2,4-oxadiazole. ¹H-NMR (CDCl₃), δ (ppm): 8.99 (s, 1H), 8.57 (s, 1H), 8.49 (d, 1H), 8.19 (s, 1H), 7.92 (d, 1H), 7:72 (t, 1H).

### 3-(2-pyridyl)-5-(5-chloro-2-methoxyphenyl)-1,2,4-oxadiazole (B37)

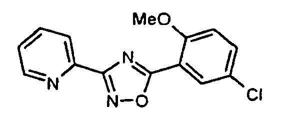

5-Chloro-O-anisic acid (187 mg, 1 mmol) was treated with a solution of oxalyl chloride (1.5 mL of 2 M in dichloromethane, 3 mmol) and a catalytic amount of *N,N*-dimethylformamide. The reaction was stirred at ambient temperature for 2 hours. The excess oxalyl chloride was removed *in vacuo* to afford 5-chloro-2-methoxybenzoyl chloride.

Using the general procedure for the synthesis of 1,2,4-oxadiazoles, the 5-chloro-2-methoxybenzoyl chloride and pyrid-2-ylamidoxime (137 mg, 1 mmol) in pyridine (1 mL) were heated at 115 °C for 17 hours. Standard work up, and silica gel chromatography afforded 49 mg (17%) of 3-(2-pyridyl)-5-(5-chloro-2-methoxyphenyl)-1,2,4-oxadiazole. ¹H-NMR (CDCl₃), δ (ppm): 4.00(s, 3H), 7.03 (d, J = 8.9 Hz, 1H), 7.42-7.47 (m, 1H), 7.50 (dd, J = 8.9 Hz, 2.8 Hz, 1H), 7.87 (ddd, J = 1.4 Hz, 7.4 Hz, 8.2 Hz, 1H), 8.22 (d, J = 8.2 Hz, 1H), 8.28 (d, J = 2.4 Hz, 1H), 8.84 (m, 1H).

### 3-(2-pyridyl)-5-(2,3-dimethoxyphenyl)-1,2,4-oxadiazole (B38)

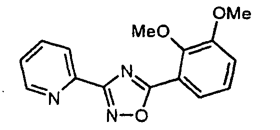

2,3-Dimethoxybenzoic acid (182 mg, 1 mmol) was treated with a solution of oxalyl chloride (1.5 mL of 2 M in dichloromethane, 3 mmol) and a catalytic amount of *N,N*-dimethylformamide. The reaction was stirred at ambient temperature for 2 hours. The excess oxalyl chloride was removed *in vacuo* to afford 2,3-dimethoxybenzoyl chloride.

Using the general procedure for the synthesis of 1,2,4-oxadiazoles, the 2,3-dimethoxybenzoyl chloride and pyrid-2-ylamidoxime (137 mg, 1 mmol) in pyridine (1 mL) were heated at 115 °C for 17 hours. Standard work up and silica gel chromatography afforded 120 mg (42%) of 3-(2-pyridyl)-5-(2,3-dimethoxyxyphenyl)-1,2,4-oxadiazole.

### 3-(2-pyridyl)-5-(2-chloro-5-methylthiophenyl)-1,2,4-oxadiazole (B39)

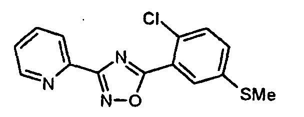

2-Chloro-5-methylthiobenzoic acid (182 mg, 1 mmol) was treated with a solution of oxalyl chloride (1.5 mL of 2 M in dichloromethane, 3 mmol) and a catalytic amount of *N,N*-dimethylformamide. The reaction was stirred at ambient temperature for 2 hours. The excess oxalyl chloride was removed *in vacuo* to afford 2-chloro-5-methylthiobenzoyl chloride.

Using the general procedure for the synthesis of 1,2,4-oxadiazoles, the 2-chloro-5-methylthiobenzoyl chloride and pyrid-2-ylamidoxime (137 mg, 1 mmol) in pyridine (1 mL) were heated at 115 °C for 17 hours. Standard work up and silica gel chromatography afforded 250 mg (82%) of 3-(2-pyridyl)-5-(2-chloro-5-methylthiophenyl)-1,2,4-oxadiazole. ¹H-NMR (CDCl₃), δ (ppm): 7.37(dd, J= 2.4Hz, 8.2 Hz, 1H), 7.40-7.50 (m, 2H), 7.89 (ddd, J = 1. 4 Hz, 7.4 Hz, 8.2 Hz, 1H), 8.05 (d, J=2.4 Hz, 1H), 8.23 (dd, J = 2.2 Hz, 8.0 Hz, 1 x H), 8.85 (m, 1H).

### 3-(2-Pyridyl)-5-(3-phenoxyphenyl)-1,2,4-oxadiazole (B40)

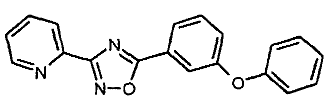

3-Phenoxybenzoic acid (214 mg, 1.0 mmol) was treated with a solution of oxalyl chloride (1.5 mL of 2 M in dichloromethane, 3 mmol) and a catalytic amount of *N,N*-dimethylformamide. The reaction was stirred overnight at ambient temperature. The excess oxalyl chloride was removed *in vacuo* to afford 3-phenoxybenzoyl chloride.

Using the general procedure for the synthesis of 1,2,4-oxadiazoles, the 3-phenoxybenzoyl chloride and pyrid-2-ylamidoxime (137 mg, 1 mmol) in pyridine (1 mL) were heated in a sealed vial overnight at 110 °C. Standard work up afforded 118 mg (37%) of 3-(2-pyridyl)-5-(3-phenoxyphenyl)-1,2,4-oxadiazole as a white solid.

### 3-(2-Pyridyl)-5-(3-benzoylphenyl)-1,2,4-oxadiazole (B41)

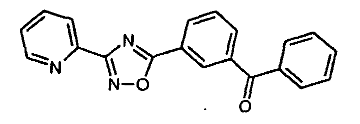

3-Benzoylbenzoic acid (226 mg, 1.0 mmol) in dichloromethane (1.5 mL) was treated with a solution of oxalyl chloride (1.5 mL of 2 M in dichloromethane, 3 mmol) and a catalytic amount of *N,N*-dimethylformamide. The reaction was stirred overnight at ambient temperature. The excess oxalyl chloride was removed *in vacuo* to afford 3-benzoylbenzoyl chloride.

Using the general procedure for the synthesis of 1,2,4-oxadiazoles, the 3-benzoylbenzoyl chloride and pyrid-2-ylamidoxime (137 mg, I mmol) in pyridine (1 mL) were heated in a sealed vial overnight at 110 °C. Standard work up and filtration through silica gel (with dichloromethane) afforded 200 mg (61%) of 3-(2-pyridyl)-5-(3-benzoylphenyl)-1,2,4-oxadiazole as a white solid. ¹H NMR (CDCl₃), δ (ppm): 8.85 (d, 1H), 8.68 (m, 1H), 8.53 (dd, 1H), 8.23 (d, 1H), 8.07 (m, 1H), 7.88 (m, 3H), 7.70 (m, 2H), 7.49 (m, 3H).

### 3-(2-Pyridyl)-5-(2-bromo-5-methoxyphenyl)-1,2,4-oxadiazole (B42)

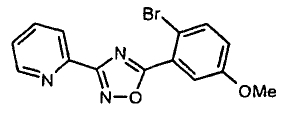

2-Bromo-5-methoxybenzoic acid (231 mg, 1.0 mmol) in dichloromethane (1.5 mL) was treated with a solution of oxalyl chloride (1.5 mL of 2 M in dichloromethane, 3 mmol) and a catalytic amount of *N,N*-dimethylformamide. The reaction was stirred overnight at ambient temperature. The excess oxalyl chloride was removed *in vacuo* to afford 2-bromo-5-methoxybenzoyl chloride.

Using the general procedure for the synthesis of 1,2,4-oxadiazoles, the 2-bromo-5-methoxybenzoyl chloride and pyrid-2-ylamidoxime (137 mg, 1 mmol) in pyridine (1 mL) were heated in a sealed vial overnight at 110 °C. Standard work up and filtration through silica gel (with dichloromethane) afforded 147 mg (44%) of 3-(2-pyridyl)-5-(2-bromo-5-methoxyphenyl)-1,2,4-oxadiazole. ¹H NMR (CDCl₃), δ (ppm): 8.85 (d, 1H), 8.24 (d, 1H), 7.89 (m, 1H), 7.65 (m, 2H), 7.47 (m, 1H), 6.99 (m, 1H), 3.89 (s, 3H).

### 3-(2-Pyridyl)-5-(2-chloro-5-(trifluoromethyl)phenyl)-1,2,4-oxadiazole (B43)

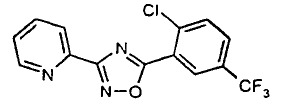

2-Chloro-5-(trifluoromethyl)benzoic acid (224 mg, 1.0 mmol) in dichloromethane (1.5 mL) was treated with a solution of oxalyl chloride (1.5 mL of 2 M in dichloromethane, 3 mmol) and a catalytic amount of *N,N*-dimethylformamide. The reaction was stirred overnight at ambient temperature. The excess oxalyl chloride was removed *in vacuo* to afford 2-chloro-5-(trifluoromethyl)benzoyl chloride.

Using the general procedure for the synthesis of 1,2,4-oxadiazoles, the 2-chloro-5-(trifluoromethyl)benzoyl chloride and pyrid-2-ylamidoxime (137 mg, 1 mmol) in pyridine (1 mL) were heated in a sealed vial overnight at 110 °C. Standard work up and filtration through silica gel (with dichloromethane) afforded 136 mg (42%) of 3-(2-pyridyl)-5-(2-chloro-5-(trifluoromethyl)phenyl)-1,2,4-oxadiazole as a beige solid. ¹H NMR (CDCl₃), δ (ppm): 8.87 (d, 1H), 8.56 (s, 1H), 8.25 (d, 1H), 7.89 (m, 1H), 7.78 (m, 2H), 7.50 (m, 1H).

### 3-(2-Pyridyl)-5-(3,4,5-trifluorophenyl)-1,2,4-oxadiazole (B44)

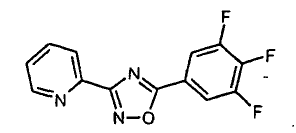

3,4,5-Trifluorobenzoic acid (0.176 g, 1.0 mmol) in dichloromethane (1.5 mL) was treated with a solution of oxalyl chloride (1.5 mL of 2 M in dichloromethane, 3 mmol) and a catalytic amount of *N,N*-dimethylformamide. The reaction was stirred overnight at ambient temperature. The excess oxalyl chloride was removed *in vacuo* to afford 3,4,5-trifluorobenzoyl chloride.

Using the general procedure for the synthesis of 1,2,4-oxadiazoles, the 3,4,5-trifluorobenzoyl chloride and pyrid-2-ylamidoxime (137 mg, 1 mmol) in pyridine (1 mL) were heated in a sealed vial overnight at 110 °C. Standard work up and silica gel chromatography (with 10-30% ethyl acetate in hexane) afforded 15 mg (5%) of 3-(2-pyridyl)-5-(3,4,5-trifluorophenyl)-1,2,4-oxadiazole as a white solid.

### 3-(2-pyridyl)-5-(2,5,6-trifluorophenyl)-1,2,4-oxadiazole (B45)

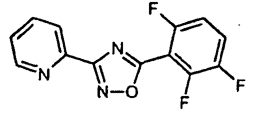

### 2,5,6-Trifluorolbenzoic acid (176 mg, 1 mmol) was treated with a solution of oxalyl chloride (1.5 mL of 2 M in dichloromethane, 3 mmol) and a catalytic amount of N,N-dimethylformamide. The reaction was stirred at ambient temperature for 16 hours. The excess oxalyl chloride was removed in vacuo to afford 2,5,6-trifluorolbenzoyl chloride.

A solution of the intermediate 2,5,6-trifluorolbenzoyl chloride and pyrid-2-ylamidoxime (137 mg, 1 mmol) in dichloromethane was stirred at ambient temperature for 0.5 hours. Silica gel chromatography afforded 151 mg (51%) of N-[(2,5,6-trifluorobenzoyl) oxy]pyridine-2-carboximidamide.

A solution of *N*-[(2,5,6- trifluorobenzoyl)oxy]pyridine-2-carboximidamide (50 mg, 0.169 mmol) in pyridine (0.3 mL) was heated at 115 °C for 17 hours. Standard work up, and silica gel chromatography, afforded 9.5 mg (20%) of 3-(2-pyridyl)-5-(2,5,6-trifluorophenyl)-1,2,4-oxadiazole.

### Example 4: Synthesis of 3,5-disubstituted-1,2,4-oxadiazoles from acylimidazoles

### 3-(3-Methoxyphenyl)-5-(2-pyridyl)-1,2,4-oxadiazole (B8)

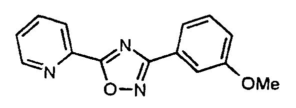

Using modifications of the method of Shine et al., *J. Heterocyclic Chem.* (1989) 26:125-128, a solution of picolinic acid (123 mg, I mmol) in pyridine (1 mL) was treated with 1,1'-carbonyldiimidazole (162 mg, 1 mmol) and the reaction stirred at ambient temperature until the evolution of carbon dioxide ceased (30 min). The intermediate acylimidazole was then treated with 3-methoxybenzamidoxime (166 mg, 1 mmol) and the reaction heated at reflux for 1 hour. Ice cold water was added to the reaction mixture to precipitate the oxadiazole. The solid was collected by filtration, washed with water and dried to afford 80 mg (32%) of 3-(3-methoxyphenyl)-5-(2-pyridyl)-1,2,4-oxadiazole: mp 90-94 °C; GC/EI-MS gave *m*/*z* (rel. int.) 253 (M⁺, 100), 254 (17), 179 (2), 175 (2), 149 (77), 133 (33), 119 (4), 106 (29). 78 (45), 51 (18).

### Example 5: Synthesis of 3,5-disubstituted-1,2,4-oxadiazoles from esters

### 3-(Pyrid-2-yl)-5-(2-hydroxyphenyl)-1,2,4-oxadiazole (B46)

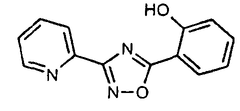

Using the method of Korbonits et al., *J. Chem. Soc. Perkin Trans. I* (1982) 759-766, a mixture of ethyl salicylate (200 mg, 1.2 mmol), pyrid-2-ylamidoxime (82.5 mg, 0.6 mmol), 21 % sodium ethoxide (19.4 mL, 6 mmol) in ethanol (12mL) was heated at reflux for 16 hours. After cooling, the reaction mixture was diluted with dichloromethane (50 mL) and washed with water and saturated sodium hydrocarbonate. The organic layer was dried with sodium sulfate and concentrated *in vacuo*. Recrystallization from diethyl ether afforded 15 mg (5%) of 3-(Pyrid-2-yl)-5-(2-hydroxyphenyl)-1,2,4-oxadiazole.

### 3-(2-pyridyl)-5-(5-chloro-2-hydroxyphenyl)-1,2,4-oxadiazole (B47)

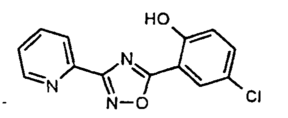

In a similar fashion, methyl 5-chloro-2-hydroxybenzoate (372 mg, 2 mmol), pyrid-2-ylamidoxime (137 mg, 1 mmol), 21 % sodium ethoxide (32.4 mL, 10 mmol) in ethanol (20 mL) were heated at reflux for 16 hours. Standard work up and recrystallization from diethyl ether afforded 14.2 mg (5%) of 3-(2-pyridyl)-5-(5-chloro-2-hydroxyphenyl)-1,2,4-oxadiazole.

### Example 6: Synthesis of 3,5-disubstituted-1,2,4-oxadiazoles from isatoic anhydrides

### 3-(2-pyridyl)-5-(2-aminophenyl)-1,2,4-oxadiazole (B48)

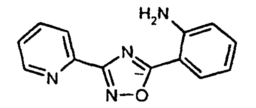

Using modifications from the procedure of Nagahara et al., *Chem. Pharm. Bull.,* (1975) 23:3178-3183, a mixture of isatoic anhydride (163 mg, 1 mmol) and pyrid-2-ylamidoxime (137 mg, 1 mmol) in pyridine (1 mL) was heated at 115 °C for 17 hours. After-cooling the reaction, the mixture was diluted with 50 mL of dichloromethane and washed with water and saturated sodium hydrocarbonate. The organic layer was dried over sodium sulfate, filtered through silica gel and concentrated *in vacuo*. Recrystallization from diethyl ether afforded 45.6 mg (19%) of 3-(2-pyridyl)-5-(2-aminophenyl)-1,2,4-oxadiazole.

### 3-(2-pyridyl)-5-(2-aminophenyl)-1,2,4-oxadiazole (B49)

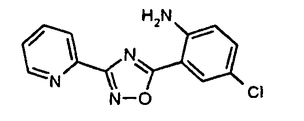

In a similar fashion, 5-chloroisatoic anhydride (197 mg, 1 mmol) and pyrid-2-ylamidoxime (137 mg, 1 mmol) in pyridine (1 mL) was heated at 115 °C for 17 hours. Work up afforded 138 mg (51%) of 3-(2-pyridyl)-5-(2-aminophenyl)-1,2,4-oxadiazole.

### Example 7: Synthesis of 2,4-disubstituted-1,3-oxazoles

### 2-[3-chlorophenyl]-4-[pyridin-2-yl]-1,3-oxazole (B50)

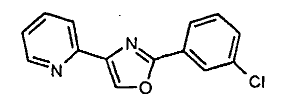

Using the procedures of Kelly et al., *J. Org. Chem.,* (1996) *61*:4623-4633, a solution of 2-bromoacetylpyridine (120 mg, 0.6 mmol) in toluene (5mL) was treated with 3-chlorobenzamide (300 mg, 1.9 mmol) and the mixture heated in a sealed vial at reflux for 60 hours. The mixture was then cooled and the solvent was removed *in vacuo*. Silica gel chromatography using a gradient of hexane to ethyl acetate afforded 38 mg (9%) of 2-[3-chlorophenyl]-4-[pyridin-2-yl]-1,3-oxazole as a pale yellow solid. ¹H-NMR (CDCl₃), δ (ppm): 8.62 (d, 1H), 8.35 (s, 1H), 8.15 (m, 1H), 8.00 (m, 2H), 7.80 (td, 1H), 7.42 (m, 2H), 7.23 (m, 1H).

### 2-[3-Bromophenyl]-4-[pyridin-2-yl]-1,3-oxazole (B51)

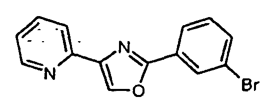

In a similar fashion 2-bromoacetylpyridine (500 mg, 2.5 mmol) and 3-chlorobenzamide (1.2g, 6 mmol) in toluene (10 mL) was heated in a sealed vial at reflux for 60 hours. Work up and silica gel chromatography using a gradient of hexane to ethyl acetate afforded 50 mg (7%) of 2-[3-bromophenyl]-4-[pyridin-2-yl]-1,3-oxazole as a white solid. ¹H-NMR (CDCl₃), δ (ppm): 8.60 (d, 1H), 8.34 (s, 1H), 8.30 (t, 1H), 8.00 (m, 2H), 7.80 (td, 1H), 7.60 (dd, 1H), 7.35 (t, 1H), 7.23 (m, 1H).

### 2-[3-cyanophenyl]-4-[pyridin-2-yl]-1,3-oxazole (B52)

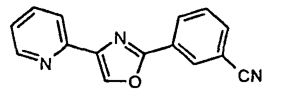

A mixture of 2-[3-bromophenyl]-4-[pyridin-2-yl]-1,3-oxazole (23 mg, 0.076 mmol) and zinc cyanide (112 mg, 0.96 mmol) in *N,N*-dimethylformamide (2 mL) was treated with Pd(PPh₃)₄ (74 mg, 0.064 mmol) and heated overnight at 80°C. Standard work up and chromatography afforded 6 mg (32 %) of 2-[3-cyanophenyl]-4-[pyridin-2-yl]-1,3-oxazole as a white solid. ¹H-NMR (CDCl₃), δ (ppm): 8.61 (d, 1H). 8.45 (s, 1H), 8.38 (s, 1H), 8.36 (m, 1H),8.00 (d, 1H), 7.80 (m, 2H), 7.61 (t, 1H), 7.23 (m, 1H).

### Example 8: Assays of Group I receptor antagonist activity

### Astrocyte Screening Assay

Primary astrocyte cultures were prepared from 3-5 day old Sprague-Dawley rat pups using a modification of Miller (Miller *et al*, *J. Neuroscience, 15(9):* 6103-6109, 1995). In brief, primary cultures were plated on poly-L lysine coated flasks in Dulbecco's modified Eagle's medium (DMEM) containing fetal calf serum (FCS). After 6 days, cell cultures were shaken over night at 280 rpm, then transferred to astrocyte-defined media (ADM) containing growth factors that up-regulate the expression of mGluR5 (Miller et al., 1995). For cuvette analysis, cultures were up-regulated with growth factors in flasks for 3-5 days, then harvested and prepared for measurement of [Ca²⁺]i mobilization as previously described (Nemeth et al., 1998).

For FLIPR analysis, cells were seeded on poly-D lysine coated clear bottom 96-well plates with black sides and analysis of [Ca²⁺]ᵢ mobilization was performed 3 days following the growth factor up-regulation. Cell cultures in the 96-well plates were loaded with a 4 µM solution of acetoxymethyl ester form of the fluorescent calcium indicator fluo-3 (Molecular Probes, Eugene, Oregon) in 0.01% pluronic. All assays were performed in a buffer containing 127 mM NaCl, 5 mM KCl, 2 mM MgCl₂, 0.7 mM NaH₂PO₄, 2 mM CaCl₂, 0.422 mg/ml NaHCO₃, 2.4 mg/ml HEPES, 1.8 mg/ml glucose and 1 mg/ml BSA Fraction IV (pH 7.4).

FLIPR experiments were done using a laser setting of 0.800 W and a 0.4 second CCD camera shutter speed. Each FLIPR experiment was initiated with 180 µL of buffer present in each well of the cell plate. A 20 µL addition from the antagonist plate was followed by a 50 µL addition from the agonist plate. After each addition the fluorescence signal was sampled 50 times at 1 second intervals followed by 3 samples at 5 second intervals. Responses were measured as the peak height of the response within the sample period.

EC₅₀/IC₅₀ determinations were made from data obtained from 8 point concentration response curves (CRC) performed in duplicate. Agonist CRC were generated by scaling all responses to the maximal response observed for the plate. Antagonist block of the agonist challenge was normalized to the average response of the agonist challenge in 14 control wells on the same plate.

### CaR/mGluR5d Screening Assay

HEK 293 cells expressing the chimeric CaR/mGluR5d receptor (clonal cell line hCaR/hmGluR5d_hek6) are plated 24 hours prior to assay at a density of 100,000 cells per well in Collagen I-coated 96-well black, clear bottom plates (Becton Dickenson) in DMEM supplemented with 10% FBS (Hyclone).

On the day of the assay, tissue culture medium is aspirated from the wells of a plate and 80 µL of Assay Buffer (Assay Buffer is: 20 mM HEPES, 146 mM NaCl, 5 mM KCl, 1 mM MgCl₂, 1 mM CaCl₂, 1 mg/ml BSA, 1 mg/ml glucose, pH 7.4) supplemented with 6 µM of the Ca²⁺-sensitive dye, Fluo-3 AM (Molecular Probes) and 0.025% Pluronic (Molecular Probes) is added to each well. The plate is then incubated in the dark for 1 hour at room temperature to efficiently load the cells with Fluo-3. At the end of the incubation, extracellular Fluo-3 is removed by washing the plate with Assay buffer. Assay Buffer is added back to each well (final volume = 160 µL) prior to beginning the assay.

The plate is loaded into a FLIPR robotic device (Molecular Devices) with the laser setting at 0.8 Watts. At a time of 10 seconds after initiation of the assay, 40 µL of Assay Buffer containing 62.5 µM test substance and 2% DMSO is added to the 160 µL of Assay Buffer in each well of the plate to yield a final concentration of 12 µM test substance and 0.4% DMSO. At a time of 75 seconds after initiation of the assay, 50 µL of Assay Buffer containing 6 mM CaCl₂ is added to the 200 µL present in each well to yield a final Ca²⁺ concentration of 2.0 mM, and a final concentration of test substance of 10 nM. Relative fluorescence intensity (excitation λ = 488 nm / emission λ = 510 nM) is monitored at relevant time intervals throughout the assay period to measure receptor activation and/or inhibition.

By way of illustration, a 1,2,4-oxadiazole disclosed above, designated "B21" (see Example 3), had an IC₅₀ value of 43 nM in relation to CaR/mGluR_{5d} and an IC₅₀ value of 121 nM on the native receptor, mGluR_{5d}. A corresponding 1,3 oxazole, designated "B52" (see Example 7), was found to be equipotent on the CaR/mGluR_{5d} chimera, with an IC₅₀ value of 45 nM, but displayed increased potency on the native mGluR_{5d} receptor, with an IC₅₀ value of 74 nM.

## Claims

1. A compound selected from
a) a compound of Formula II: 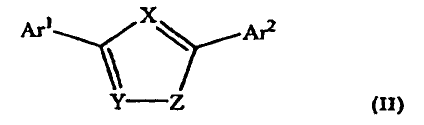 wherein X and Y are N,
Z is O;
Ar¹ is 2-pyridyl and Ar² is phenyl, wherein at least one of the Ar¹ and Ar² moieties are substituted with one or more moieties selected from the group consisting of -F, -Cl, -Br, -I, -SR, -SOR, -SO₂R, -SO₂NRR', -OCOR, -OCONRR', -NRCOR', -NRCO₂R', -CN, -CO₂R, - CONRR', -C(O)R, -CH(OR)R', -CH₂(OR), and -R;
wherein R or R' is selected from the group consisting of H, CF₃, C₁-C₁₀ alkyl, cycloalkyl, alkyl-aryl, heterocycloalkyl, aryl and where R and R' may combine to form a ring,
with the proviso that the compound is not 3-(2-pyridyl)-5-(2-chlorophenyl)-1,2,4-oxadiazole 3-(2-pyridyl)-5-phenyl-1,2,4-oxadiazole, or 3-(2-pyridyl)-5-[3-(trifluoromethyl)phenyl]-1,2,4-oxadiazole; or
b) 3-(2-pyridyl)-5-[3-(trifluoromethoxy)phenyl]-1,2,4-oxadiazole,
3-(2-pyridyl)-5-(2,3-difluorophenyl)-1,2,4-oxadiazole,
3-(2-pyridyl)-5-(3,5-dimethoxyphenyl)-1,2,4-oxadiazole,
3-(5-fluoropyrid-2-yl)-5-(3,5-dimethoxyphenyl)-1,2,4-oxadiazole,
3-(5-methoxypyrid-2-yl)-5-(3-cyanophenyl)-1,2,4-oxadiazole,
3-(2-pyridyl)-5-(3-nitrophenyl)-1,2,4-oxadiazole,
2-[3-chlorophenyl]-4-[pyridin-2-yl]-1,3-oxazole,
2-(3,5-dichlorophenyl)-4-(2-pyridyl)-1,3-oxazole,
2-(3-chlorophenyl)-4-(2-pyridyl)-1,3-oxazole,
2-(3-methoxyphenyl)-4-(2-pyridyl)-1,3-oxazole,
2-(2-chlorophenyl)-4-(2-pyridyl)-1,3-oxazole,
2-(3-trifluorophenyl)-4-(2-pyridyl)-1,3-oxazole,
2-(3-methylphenyl)-4-(2-pyridyl)-1,3-oxazole,
2-(3-trifluoromethoxyphenyl)-4-(2-pyridyl)-1,3-oxazole,
2-(2,3-difluorophenyl)-4-(2-pyridyl)-1,3-oxazole,
2-(2,5-difluorophenyl)-4-(2-pyridyl)-1,3-oxazole,
2-(3,5-difluorophenyl)-4-(2-pyridyl)-1,3-oxazole,
2-(3-cyanophenyl)-4-(2-pyridyl)-1,3-oxazole,
2-(3,5-dimethoxyphenyl)-4-(2-pyridyl)-1,3-oxazole,
2-(2,3-dichlorophenyl)-4-(2-pyridyl)-1,3-oxazole,
2-(3-chloro-5-cyanophenyl)-4-(2-pyridyl)-1,3-oxazole,
2-(3-fluoro-5-cyanophenyl)-4-(2-pyridyl)-1,3-oxazole,
2-(3-chloro-5-fluorophenyl)-4-(2-pyridyl)-1,3-oxazole,
2-(3-cyanophenyl)-4-(5-chloropyrid-2-yl)-1,3-oxazole,
2-(3-cyanophenyl)-4-(5-fluoropyrid-2-yl)-1,3-oxazole,
2-(3-cyano-5-fluorophenyl)-4-(5-fluoropyrid-2-yl)-1,3-oxazole,
2-(3-cyanophenyl)-4-(3-fluoropyrid-2-yl)-1,3-oxazole,
2-(3,5-dimethoxyphenyl)-4-(5-fluoropyrid-2-yl)-1,3-oxazole,
2-(3-cyanophenyl)-4-(5-methoxypyrid-2-yl)-1,3-oxazole,
2-(3-cyanophenyl)-4-(3-chloro-5-trifluoromethylpyrid-2-yl)-1,3-oxazole,
2-(5-chloro-2-methoxyphenyl)-4-(2-pyridyl)-1,3-oxazole,
2-(2-chloro-5-methylthlophenyl)-4-(2-pyridyl)-1,3-oxazole,
2-(2-bromo-5-methoxyphenyl)-4-(2-pyridyl)-1,3-oxazole,
2-(2,5,6-trifluorophenyl)-4-(2-pyridyl)-1,3-oxazole,
2-[3-chlorophenyl]-4-[pyridin-2-yl]-1,3-oxazole,
2-(2,5,6-trifluorophenyl)-4-(2-pyridyl)-1,3-oxazole,
2-(3-nitrophenyl)-4-(2-pyridyl)-1,3-oxazole,
2-(3-bromophenyl)-4-(2-pyridyl)-1,3-oxazole, or
a pharmaceutically acceptable salt thereof.

2. The compound of claim 1, wherein Ar² is substituted with one or more moieties selected from the group consisting of CF₃, Cl, F, Br, CH₃, SCH₃, and CN.

3. The compound of claim 1, wherein Ar¹ is substituted with one or more moieties selected from the group consisting of CF₃, F, and Cl.

4. A compound of claim 1, said compound being selected from the group consisting of 3-(2-pyridyl)-5-(3,5-dichlorophenyl)-1,2,4-oxadiazole, 3-(2-pyridyl)-5-(3-chlorophenyl)-1,2,4-oxadiazole, 3-(2-pyridyl)-5-(3-methylphenyl)-1,2,4-oxadiazole, 3-(2-pyridyl)-5-(2,5-difluorophenyl)-1,2,4-oxadiazole, 3-(2-pyridyl)-5-(3,5-difluorophenyl)-1,2,4-oxadiazole, 3-(2-pyridyl)-5-(3-cyanophenyl)-1,2,4-oxadiazole, 3-(2-pyridyl)-5-(2,3-dichlorophenyl)-1,2,4-oxadiazole, 3-(2-pyridyl)-5-(3-chloro-5-cyanophenyl)-1,2,4-oxadiazole, 3-(2-pyridyl)-5-(3-fluoro-5-cyanophenyl)-1,2,4-oxadiazole, 3-(2-pyridyl)-5-(3-chloro-5-fluorophenyl)-1,2,4-oxadiazole, 3-(5-chloropyrid-2-yl)-5-(3-cyanophenyl)-1,2,4-oxadiazole, 3-(5-fluoropyrid-2-yl)-5-(3-cyanophenyl)-1,2,4-oxadiazole, 3-(5-fluoropyrid-2-yl)-5-(3-cyano-5-fluorophenyl)-1,2,4-oxadiazole, 3-(3-fluoropyrid-2-yl)-5-(3-cyanophenyl)-1,2,4-oxadiazole, 3-(3-chloro-5-trifluoromethylpyrid-2-yl)-5-(3-cyanophenyl)-1,2,4-oxadiazole, 3-(2-pyridyl)-5-(5-chloro-2-methoxyphenyl)-1,2,4-oxadiazole, 3-(2-pyridyl)-5-(2-chloro-5-methylthiophenyl)-1,2,4-oxadiazole, 3-(2-pyridyl)-5-(2,5,6-trifluorophenyl)-1,2,4-oxadiazole, 3-(2-pyridyl)-5-(2,5,6-trifluorophenyl)-1,2,4-oxadiazole, 3-(2-pyridyl)-5-(3-bromophenyl)-1,2,4-oxadiazole and pharmaceutically acceptable salts thereof.

5. A pharmaceutical composition comprising a compound selected from:
a) a compound of Formula II: 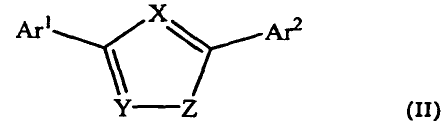 wherein X and Y are N,
Z is O;
Ar¹ is 2-pyridyl and Ar² is phenyl, and wherein at least one of the Ar¹ and Ar² moieties are substituted with one or more moieties selected from the group consisting of -F, -Cl, -Br, -I, -SR, -SOR, -SO₂R, -SO₂NRR', -OCOR, -OCONRR', -NRCOR', -NRCO₂R', -CN, -CO₂R, - CONRR', -C(O)R, -CH(OR)R', -CH₂(OR), and -R;
wherein R or R' is selected from the group consisting of H, CF₃, C₁-C₁₀ alkyl, cycloalkyl, alkyl-aryl, heterocycloalkyl, aryl and where R and R' may combine to form a ring,
with the proviso that the compound is not 3-(2-pyridyl)-5-(2-chlorophenyl)-1,2,4-oxadiazole, or 3-(2-pyridyl)-5-[3-(trifluoromethyl)phenyl]-1,2,4-oxadiazole; or
b) 3-(2-pyridyl)-5-[3-(trifluoromethoxy)phenyl]-1,2,4-oxadiazole,
3-(2-pyridyl)-5-(2,3-difluorophenyl)-1,2,4-oxadiazole,
3-(2-pyridyl)-5-(3,5-dimethoxyphenyl)-1,2,4-oxadiazole,
3-(5-fluoropyrid-2-yl)-5-(3,5-dimethoxyphenyl)-1,2,4-oxadiazole,
3-(5-methoxypyrid-2-yl)-5-(3-cyanophenyl)-1,2,4-oxadiazole,
3-(2-pyridyl)-5-(3-nitrophenyl)-1,2,4-oxadiazole,
2-[3-chlorophenyl]-4-[pyridin-2-yl]-1,3-oxazole,
2-(3,5-dichlorophenyl)-4-(2-pyridyl)-1,3-oxazole,
2-(3-chlorophenyl)-4-(2-pyridyl)-1,3-oxazole,
2-(3-methoxyphenyl)-4-(2-pyridyl)-1,3-oxazole,
2-(2-chlorophenyl)-4-(2-pyridyl)-1,3-oxazole,
2-(3-trifluorophenyl)-4-(2-pyridyl)-1,3-oxazole,
2-(3-methylphenyl)-4-(2-pyridyl)-1,3-oxazole,
2-(3-trifluoromethoxyphenyl)-4-(2-pyridyl)-1,3-oxazole,
2-(2,3-difluorophenyl)-4-(2-pyridyl)-1,3-oxazole,
2-(2,5-difluorophenyl)-4-(2-pyridyl)-1,3-oxazole,
2-(3,5-difluorophenyl)-4-(2-pyridyl)-1,3-oxazole,
2-(3-cyanophenyl)-4-(2-pyridyl)-1,3-oxazole,
2-(3,5-dimethoxyphenyl)-4-(2-pyridyl)-1,3-oxazole,
2-(2,3-dichlorophenyl)-4-(2-pyridyl)-1,3-oxazole,
2-(3-chloro-5-cyanophenyl)-4-(2-pyridyl)-1,3-oxazole,
2-(3-fluoro-5-cyanophenyl)-4-(2-pyridyl)-1,3-oxazole,
2-(3-chloro-5-fluorophenyl)-4-(2-pyridyl)-1,3-oxazole,
2-(3-cyanophenyl)-4-(5-chloropyrid-2-yl)-1,3-oxazole,
2-(3-cyanophenyl)-4-(5-fluoropyrid-2-yl)-1,3-oxazole,
2-(3-cyano-5-fluorophenyl)-4-(5-fluoropyrid-2-yl)-1,3-oxazole,
2-(3-cyanophenyl)-4-(3-fluoropyrid-2-yl)-1,3-oxazole,
2-(3,5-dimethoxyphenyl)-4-(5-fluoropyrid-2-yl)-1,3-oxazole,
2-(3-cyanophenyl)-4-(5-methoxypyrid-2-yl)-1,3-oxazole,
2-(3-cyanophenyl)-4-(3-chloro-5-trifluoromethylpyrid-2-yl)-1,3-oxazole,
2-(5-chloro-2-methoxyphenyl)-4-(2-pyridyl)-1,3-oxazole,
2-(2-chloro-5-methylthiophenyl)-4-(2-pyridyl)-1,3-oxazole,
2-(2-bromo-5-methoxyphenyl)-4-(2-pyridyl)-1,3-oxazole,
2-(2,5,6-trifluorophenyl)-4-(2-pyridyl)-1,3-oxazole,
2-[3-chlorophenyl]-4-[pyridin-2-yl]-1,3-oxazole,
2-(2,5,6-trifluorophenyl)-4-(2-pyridyl)-1,3-oxazole,
2-(3-nitrophenyl)-4-(2-pyridyl)-1,3-oxazole,
2-(3-bromophenyl)-4-(2-pyridyl)-1,3-oxazole, or
a pharmaceutically acceptable salt thereof;
and a pharmaceutically acceptable excipient.

6. The composition of claim 5, wherein Ar² is substituted with one or more moieties selected from the group consisting of CF₃, Cl, F, Br, CH₃, CN, and SCH₃.

7. The composition of claim 5, wherein Ar¹ is substituted with one or more moieties selected from the group consisting of CF₃, F, and Cl.

8. Use of a pharmaceutically acceptable amount of a compound selected from:
a) a compound of Formula II: 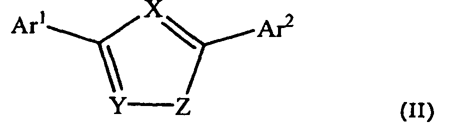 wherein X and Y are N,
Z is O;
Ar¹ is 2-pyridyl and Ar² is phenyl, and wherein at least one of the Ar¹ and Ar² moieties are substituted with one or more moieties selected from the group consisting of -F, -Cl, -Br, -I, -SR, -SOR, -SO₂R, -SO₂NRR', -OCOR, -OCONRR', -NRCOR', -NRCO₂R', -CN, -CO₂R, -CONRR', -C(O)R, -CH(OR)R', -CH₂(OR), and -R;
wherein R or R' is selected from the group consisting of H, CF₃, C₁-C₁₀ alkyl, cycloalkyl, alkyl-aryl, heterocycloalkyl, aryl and where R and R' may combine to form a ring,
with the proviso that the compound is not 3-(2-pyridyl)-5-(2-chlorophenyl)-1,2,4-oxadiazole, or 3-(2-pyridyl)-5-[3-(trifluoromethyl)phenyl]-1,2,4-oxadiazole; or
b) 3-(2-pyridyl)-5-[3-(trifluoromethoxy)phenyl]-1,2,4-oxadiazole,
3-(2-pyridyl)-5-(2,3-difluorophenyl)-1,2,4-oxadiazole,
3-(2-pyridyl)-5-(3,5-dimethoxyphenyl)-1,2,4-oxadiazole,
3-(5-fluoropyrid-2-yl)-5-(3,5-dimethoxyphenyl)-1,2,4-oxadiazole,
3-(5-methoxypyrid-2-yl)-5-(3-cyanophenyl)-1,2,4-oxadiazole,
3-(2-pyridyl)-5-(3-nitrophenyl)-1,2,4-oxadiazole,
2-[3-chlorophenyl]-4-[pyridin-2-yl]-1,3-oxazole,
2-(3,5-dichlorophenyl)-4-(2-pyridyl)-1,3-oxazole,
2-(3-chlorophenyl)-4-(2-pyridyl)-1,3-oxazole,
2-(3-methoxyphenyl)-4-(2-pyridyl)-1,3-oxazole,
2-(2-chlorophenyl)-4-(2-pyridyl)-1,3-oxazole,
2-(3-trifluorophenyl)-4-(2-pyridyl)-1,3-oxazole,
2-(3-methylphenyl)-4-(2-pyridyl)-1,3-oxazole,
2-(3-trifluoromethoxyphenyl)-4-(2-pyridyl)-1,3-oxazole,
2-(2,3-difluorophenyl)-4-(2-pyridyl)-1,3-oxazole,
2-(2,5-difluorophenyl)-4-(2-pyridyl)-1,3-oxazole,
2-(3,5-difluorophenyl)-4-(2-pyridyl)-1,3-oxazole,
2-(3-cyanophenyl)-4-(2-pyridyl)-1,3-oxazole,
2-(3,5-dimethoxyphenyl)-4-(2-pyridyl)-1,3-oxazole,
2-(2,3-dichlorophenyl)-4-(2-pyridyl)-1,3-oxazole,
2-(3-chloro-5-cyanophenyl)-4-(2-pyridyl)-1,3-oxazole,
2-(3-fluoro-5-cyanophenyl)-4-(2-pyridyl)-1,3-oxazole,
2-(3-chloro-5-fluorophenyl)-4-(2-pyridyl)-1,3-oxazole,
2-(3-cyanophenyl)-4-(5-chloropyrid-2-yl)-1,3-oxazole,
2-(3-cyanophenyl)-4-(5-fluoropyrid-2-yl)-1,3-oxazole,
2-(3-cyano-5-fluorophenyl)-4-(5-fluoropyrid-2-yl)-1,3-oxazole,
2-(3-cyanophenyl)-4-(3-fluoropyrid-2-yl)-1,3-oxazole,
2-(3,5-dimethoxyphenyl)-4-(5-fluoropyrid-2-yl)-1,3-oxazole,
2-(3-cyanophenyl)-4-(5-methoxypyrid-2-yl)-1,3-oxazole,
2-(3-cyanophenyl)-4-(3-chloro-5-trifluoromethylpyrid-2-yl)-1,3-oxazole,
2-(5-chloro-2-methoxyphenyl)-4-(2-pyridyl)-1,3-oxazole,
2-(2-chloro-5-methylthiophenyl)-4-(2-pyridyl)-1,3-oxazole,
2-(2-bromo-5-methoxyphenyl)-4-(2-pyridyl)-1,3-oxazole,
2-(2,5,6-trifluorophenyl)-4-(2-pyridyl)-1,3-oxazole,
2-[3-chlorophenyl]-4-[pyridin-2-yl]-1,3-oxazole,
2-(2,5,6-trifluorophenyl)-4-(2-pyridyl)-1,3-oxazole,
2-(3-nitrophenyl)-4-(2-pyridyl)-1,3-oxazole, or
2-(3-bromophenyl)-4-(2-pyridyl)-1,3-oxazole;
with the proviso that the compound is not 3-(2-Pyridyl)-5-(2-chlorophenyl)-1,2,4-oxadiazole, for the preparation of a medicament for the treatment of diseases associated with metabotropic glutamate receptors.

9. The use according to claim 8, wherein Ar² is substituted with one or more moieties selected from the group consisting of CF₃, Cl, F, Br, CH₃, CN, and SCH₃.

10. The use according to claim 10, wherein Ar¹ and substituted with one or more moieties selected from the group consisting of CF₃, F, and Cl.

11. The use according to claim 8, wherein the disease associated with metabotropic glutamate receptors is a neurological disease or disorder.

12. The use according to claim 8, wherein the disease associated with metabotropic glutamate receptors is a psychiatric disease.

13. The use according to claim 8, wherein the disease or disorder is selected from the group consisting of stroke, head trauma, anoxic injury, ischemic injury, hypoglycemia, epilepsy, pain, migraine headaches, Parkinson's disease, senile dementia, Huntington's Chorea and Alzheimer's disease.

14. The use according to claim 8, wherein the disease or disorder is selected from the group consisting of schizophrenia and depression.

## Patentansprüche

1. Verbindung, ausgewählt aus
a) einer Verbindung der Formel II: 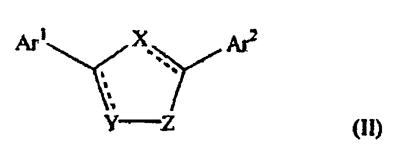 worin X und Y für N stehen,
Z für O steht;
Ar¹ für 2-Pyridyl steht und Ar² für Phenyl steht, wobei mindestens eine der Ar¹- und Ar²-Gruppierungen mit einer oder mehreren Gruppierungen, ausgewählt aus der Gruppe, bestehend aus -F, -Cl, -Br, -I, -SR, -SOR, -SO₂R, -SO₂NRR', -OCOR, -OCONRR', -NRCOR', -NRCO₂R', -CN, -CO₂R, -CONRR', -C(O)R, -CH(OR)R', -CH₂(OR) und -R, substituiert ist;
worin R oder R' ausgewählt ist aus der Gruppe, bestehend aus H, CF₃, C₁-C₁₀-Alkyl, Cycloalkyl, Alkylaryl, Heterocycloalkyl, Aryl, und worin R und R' unter Bildung eines Rings kombiniert sein können,
unter der Maßgabe, dass die Verbindung nicht 3-(2-Pyridyl)-5-(2-Chlorphenyl)-1,2,4-oxadiazol oder 3-(2-Pyridyl)-5-[3-(trifluormethyl)phenyl]-1,2,4-oxadiazol ist; oder
b) 3-(2-Pyridyl)-5-[3-(trifluormethoxy)phenyl]-1,2,4-oxadiazol,
3-(2-Pyridyl)-5-(2,3-difluorphenyl)-1,2,4-oxadiazol,
3-(2-Pyridyl)-5-(3,5-dimethoxyphenyl)-1,2,4-oxadiazol,
3-(5-Fluorpyrid-2-yl)-5-(3,5-dimethoxyphenyl)-1,2,4-oxadiazol,
3-(5-Methoxypyrid-2-yl)-5-(3-cyanophenyl)-1,2,4-oxadiazol,
3-(2-Pyridyl)-5-(3-nitrophenyl)-1,2,4-oxadiazol,
2-[3-Chlorphenyl]-4-[pyridin-2-yl]-1,3-oxazol,
2-(3,5-Dichlorphenyl)-4-(2-pyridyl)-1,3-oxazol,
2-(3-Chlorphenyl)-4-(2-pyridyl)-1,3-oxazol,
2-(3-Methoxyphenyl)-4-(2-pyridyl)-1,3-oxazol,
2-(2-Chlorphenyl)-4-(2-pyridyl)-1,3-oxazol,
2-(3-Trifluorphenyl)-4-(2-pyridyl)-1,3-oxazol,
2-(3-Methylphenyl)-4-(2-pyridyl)-1,3-oxazol,
2-(3-Trifluormethoxyphenyl)-4-(2-pyridyl)-1,3-oxazol,
2-(2,3-Difluorphenyl)-4-(2-pyridyl)-1,3-oxazol,
2-(2,5-Difluorphenyl)-4-(2-pyridyl)-1,3-oxazol,
2-(3,5-Difluorphenyl)-4-(2-pyridyl)-1,3-oxazol,
2-(3-Cyanophenyl)-4-(2-pyridyl)-1,3-oxazol,
2-(3,5-Dimethoxyphenyl)-4-(2-pyridyl)-1,3-oxazol,
2-(2,3-Dichlorphenyl)-4-(2-pyridyl)-1,3-oxazol,
2-(3-Chlor-5-cyanophenyl)-4-(2-pyridyl)-1,3-oxazol,
2-(3-Fluor-5-cyanophenyl)-4-(2-pyridyl)-1,3-oxazol,
2-(3-Chlor-5-fluorphenyl)-4-(2-pyridyl)-1,3-oxazol,
2-(3-Cyanophenyl)-4-(5-chlorpyrid-2-yl)-1,3-oxazol,
2-(3-Cyanophenyl)-4-(5-fluorpyrid-2-yl)-1,3-oxazol,
2-(3-Cyano-5-fluorphenyl)-4-(5-fluorpyrid-2-yl)-1,3-oxazol,
2-(3-Cyanophenyl)-4-(3-fluorpyrid-2-yl)-1,3-oxazol,
2-(3,5-Dimethoxyphenyl)-4-(5-fluorpyrid-2-yl)-1,3-oxazol,
2-(3-Cyanophenyl)-4-(5-methoxypyrid-2-yl)-1,3-oxazol,
2-(3-Cyanophenyl)-4-(3-chlor-5-trifluormethylpyrid-2-yl)-1,3-oxazol,
2-(5-Chlor-2-methoxyphenyl)-4-(2-pyridyl)-1,3-oxazol,
2-(2-Chlor-5-methylthiophenyl)-4-(2-pyridyl)-1,3-oxazol,
2-(2-Brom-5-methoxyphenyl)-4-(2-pyridyl)-1,3-oxazol,
2-(2,5,6-Trifluorphenyl)-4-(2-pyridyl)-1,3-oxazol,
2-[3-Chlorphenyl]-4-[pyridin-2-yl]-1,3-oxazol,
2-(2,5,6-Trifluorphenyl)-4-(2-pyridyl)-1,3-oxazol,
2-(3-Nitrophenyl)-9-(2-pyridyl)-1,3-oxazol,
2-(3-Bromphenyl)-9-(2-pyridyl)-1,3-oxazol oder
ein pharmazeutisch annehmbares Salz davon.

2. Verbindung nach Anspruch 1, worin Ar² mit einer oder mehreren Gruppierungen, ausgewählt aus der Gruppe, bestehend aus CF₃, Cl, F, Br, CH₃, SCH₃ und CN substituiert ist.

3. Verbindung nach Anspruch 1, worin Ar¹ mit einer oder mehreren Gruppierungen, ausgewählt aus der Gruppe, bestehend aus CF₃, F und Cl, substituiert ist.

4. Verbindung nach Anspruch 1, wobei die Verbindung ausgewählt ist aus der Gruppe, bestehend aus 3-(2-Pyridyl)-5-(3,5-dichlorphenyl)-1,2,4-oxadiazol, 3-(2-Pyridyl)-5-(3-chlorphenyl)-1,2,4-oxadiazol, 3-(2-Pyridyl)-5-(3-methylphenyl)-1,2,4-oxadiazol, 3-(2-Pyrdiyl)-5-(2,5-difluorphenyl)-1,2,4-oxadiazol, 3-(2-Pyridyl)-5-(3,5-difluorphenyl)-1,2,4-oxadiazol, 3-(2-Pyridyl)-5-(3-cyanophenyl)-1,2,4-oxadiazol, 3-(2-Pyridyl)-5-(2,3-dichlorphenyl)-1,2,4-oxadiazol, 3-(2-Pyridyl)-5-(3-chlor-5-cyanophenyl)-1,2,4-oxadiazol, 3-(2-Pyridyl)-5-(3-fluor-5-cyanophenyl)-1,2,4-oxadiazol, 3-(2-Pyridyl)-5-(3-chlor-5-fluorphenyl)-1,2,4-oxadiazol, 3-(5-Chlorpyrid-2-yl)-5-(3-cyanophenyl)-1,2,4-oxadiazol, 3-(5-Fluorpyrid-2-yl)-5-(3-cyanophenyl)-1,2,4-oxadiazol, 3-(5-Fluorpyrid-2-yl)-5-(3-cyano-5-fluorphenyl)-1,2,4-oxadiazol, 3-(3-Fluorpyrid-2-yl)-5-(3-cyanophenyl)-1,2,4-oxadiazol, 3-(3-Chlor-5-trifluormethylpyrid-2-yl)-5-(3-cyanophenyl)-1,2,4-oxadiazol, 3-(2-Pyridyl)-5-(5-chlor-2-methoxyphenyl)-1,2,4-oxadiazol, 3-(2-Pyridyl)-5-(2-chlor-5-methylthiophenyl)-1,2,4-oxadiazol, 3-(2-Pyridyl)-5-(2,5,6-trifluorphenyl)-1,2,4-oxadiazol, 3-(2-Pyridyl)-5-(2,5,6-trifluorphenyl)-1,2,4-oxadiazol, 3-(2-Pyridyl)-5-(3-bromphenyl)-1,2,4-oxadiazol und pharmazeutisch annehmbaren Salzen davon.

5. Pharmazeutische Zusammensetzung, umfassend eine Verbindung, ausgewählt aus:
a) einer Verbindung der Formel II: 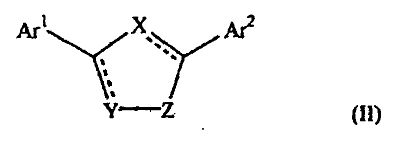 worin X und Y für N stehen,
Z für O steht;
Ar¹ für 2-Pyridyl und Ar² für Phenyl steht und worin mindestens eine der Ar¹- und Ar²-Gruppierungen mit einer oder mehreren Gruppierungen, ausgewählt aus der Gruppe, bestehend aus -F, -Cl, -Br, -I, -SR, -SOR, -SO₂R, -SO₂NRR', -OCOR, -OCONRR', -NRCOR', -NRCO₂R', -CN, -CO₂R, -CONRR', -C(O)R, -CH(OR)R', -CH₂(OR) und -R, substituiert ist;
worin R oder R' ausgewählt ist aus der Gruppe, bestehend aus H, CF₃, C₁-C₁₀-Alkyl, Cycloalkyl, Alkylaryl, Heterocycloalkyl, Aryl, und worin R und R' unter Bildung eines Rings kombiniert sein können,
unter der Maßgabe, dass die Verbindung nicht 3-(2-Pyridyl)-5-(2-Chlorphenyl)-1,2,4-oxadiazol oder 3-(2-Pyridyl)-5-[3-(trifluormethyl)phenyl]-1,2,4-oxadiazol ist; oder
b) 3-(2-Pyridyl)-5-[3-(trifluormethoxy)phenyl]-1,2,4-oxadiazol,
3-(2-Pyridyl)-5-(2,3-difluorphenyl)-1,2,4-oxadiazol,
3-(2-Pyridyl)-5-(3,5-dimethoxyphenyl)-1,2,4-oxadiazol,
3-(5-Fluorpyrid-2-yl)-5-(3,5-dimethoxyphenyl)-1,2,4-oxadiazol,
3-(5-Methoxypyrid-2-yl)-5-(3-cyanophenyl)-1,2,4-oxadiazol,
3-(2-Pyridyl)-5-(3-nitrophenyl)-1,2,4-oxadiazol,
2-[3-Chlorphenyl]-4-[pyridin-2-yl]-1,3-oxazol,
2-(3,5-Dichlorphenyl)-4-(2-pyridyl)-1,3-oxazol,
2-(3-Chlorphenyl)-4-(2-pyridyl)-1,3-oxazol,
2-(3-Methoxyphenyl)-4-(2-pyridyl)-1,3-oxazol,
2-(2-Chlorphenyl)-4-(2-pyridyl)-1,3-oxazol,
2-(3-Trifluorphenyl)-4-(2-pyridyl)-1,3-oxazol,
2-(3-Methylphenyl)-4-(2-pyridyl)-1,3-oxazol,
2-(3-Trifluormethoxyphenyl)-4-(2-pyridyl)-1,3-oxazol,
2-(2,3-Difluorphenyl)-4-(2-pyridyl)-1,3-oxazol,
2-(2,5-Difluorphenyl)-4-(2-pyridyl)-1,3-oxazol,
2-(3,5-Difluorphenyl)-4-(2-pyridyl)-1,3-oxazol,
2-(3-Cyanophenyl)-4-(2-pyridyl)-1,3-oxazol,
2-(3,5-Dimethoxyphenyl)-4-(2-pyridyl)-1,3-oxazol,
2-(2,3-Dichlorphenyl)-4-(2-pyridyl)-1,3-oxazol,
2-(3-Chlor-5-cyanophenyl)-4-(2-pyridyl)-1,3-oxazol,
2-(3-Fluor-5-cyanophenyl)-4-(2-pyridyl)-1,3-oxazol,
2-(3-Chlor-5-fluorphenyl)-4-(2-pyridyl)-1,3-oxazol,
2-(3-Cyanophenyl)-4-(5-chlorpyrid-2-yl)-1,3-oxazol,
2-(3-Cyanophenyl)-4-(5-fluorpyrid-2-yl)-1,3-oxazol,
2-(3-Cyano-5-fluorphenyl)-4-(5-fluorpyrid-2-yl)-1,3-oxazol,
2-(3-Cyanophenyl)-4-(3-fluorpyrid-2-yl)-1,3-oxazol,
2-(3,5-Dimethoxyphenyl)-4-(5-fluorpyrid-2-yl)-1,3-oxazol,
2-(3-Cyanophenyl)-4-(5-methoxypyrid-2-yl)-1,3-oxazol,
2-(3-Cyanophenyl)-4-(3-chlor-5-trifluormethylpyrid-2-yl)-1,3-oxazol,
2-(5-Chlor-2-methoxyphenyl)-4-(2-pyridyl)-1,3-oxazol,
2-(2-Chlor-5-methylthiophenyl)-4-(2-pyridyl)-1,3-oxazol,
2-(2-Brom-5-methoxyphenyl)-4-(2-pyridyl)-1,3-oxazol,
2-(2,5,6-Trifluorphenyl)-4-(2-pyridyl)-1,3-oxazol,
2-[3-Chlorphenyl]-4-[pyridin-2-yl]-1,3-oxazol,
2-(2,5,6-Trifluorphenyl)-4-(2-pyridyl)-1,3-oxazol,
2-(3-Nitrophenyl)-4-(2-pyridyl)-1,3-oxazol,
2-(3-Bromphenyl)-4-(2-pyridyl)-1,3-oxazol oder
ein pharmazeutisch annehmbares Salz davon;
und ein pharmazeutisch annehmbares Exzipiens.

6. Zusammensetzung nach Anspruch 5, worin Ar² mit einer oder mehreren Gruppierungen, ausgewählt aus der Gruppe, bestehend aus CF₃, Cl, F, Br, CH₃, CN und SCH₃, substituiert ist.

7. Zusammensetzung nach Anspruch 5, worin Ar¹ mit einer oder mehreren Gruppierungen, ausgewählt aus der Gruppe, bestehend aus CF₃, F und Cl, substituiert ist.

8. Verwendung einer pharmazeutisch annehmbaren Menge einer Verbindung, ausgewählt aus:
a) einer Verbindung der Formel II: 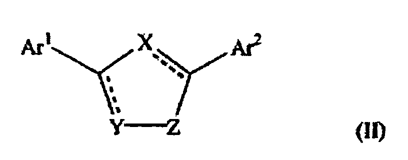 worin X und Y für N stehen,
Z für O steht;
Ar¹ für 2-Pyridyl und Ar² für Phenyl steht und worin mindestens eine der Ar¹- und Ar²-Gruppierungen mit einer oder mehreren Gruppierungen, ausgewählt aus der Gruppe, bestehend aus -F, -Cl, -Br, -I, -SR, -SOR, -SO₂R, -SO₂NRR', -OCOR, -OCONRR', -NRCOR', -NRCO₂R', -CN, -CO₂R, -CONRR', -C(O)R, -CH(OR)R', -CH₂(OR) und -R, substituiert ist;
worin R oder R' ausgewählt ist aus der Gruppe, bestehend aus H, CF₃, C₁-C₁₀-Alkyl, Cycloalkyl, Alkylaryl, Heterocycloalkyl, Aryl, und worin R und R' unter Bildung eines Rings kombiniert sein können,
unter der Maßgabe, dass die Verbindung nicht 3-(2-Pyridyl)-5-(2-Chlorphenyl)-1,2,4-oxadiazol oder 3-(2-Pyridyl)-5-[3-(trifluormethyl)phenyl]-1,2,4-oxadiazol ist; oder
b) 3-(2-Pyridyl)-5-[3-(trifluormethoxy)phenyl]-1,2,4-oxadiazol,
3-(2-Pyridyl)-5-(2,3-difluorphenyl)-1,2,4-oxadiazol,
3-(2-Pyridyl)-5-(3,5-dimethoxyphenyl)-1,2,4-oxadiazol,
3-(5-Fluorpyrid-2-yl)-5-(3,5-dimethoxyphenyl)-1,2,4-oxadiazol,
3-(5-Methoxypyrid-2-yl)-5-(3-cyanophenyl)-1,2,4-oxadiazol,
3-(2-Pyridyl)-5-(3-nitrophenyl)-1,2,4-oxadiazol,
2-[3-Chlorphenyl]-4-[pyridin-2-yl]-1,3-oxazol,
2-(3,5-Dichlorphenyl)-4-(2-pyridyl)-1,3-oxazol,
2-(3-Chlorphenyl)-4-(2-pyridyl)-1,3-oxazol,
2-(3-Methoxyphenyl)-4-(2-pyridyl)-1,3-oxazol,
2-(2-Chlorphenyl)-4-(2-pyridyl)-1,3-oxazol,
2-(3-Trifluorphenyl)-4-(2-pyridyl)-1,3-oxazol,
2-(3-Methylphenyl)-4-(2-pyridyl)-1,3-oxazol,
2-(3-Trifluormethoxyphenyl)-4-(2-pyridyl)-1,3-oxazol,
2-(2,3-Difluorphenyl)-4-(2-pyridyl)-1,3-oxazol,
2-(2,5-Difluorphenyl)-4-(2-pyridyl)-1,3-oxazol,
2-(3,5-Difluorphenyl)-4-(2-pyridyl)-1,3-oxazol,
2-(3-Cyanophenyl)-4-(2-pyridyl)-1,3-oxazol,
2-(3,5-Dimethoxyphenyl)-4-(2-pyridyl)-1,3-oxazol,
2-(2,3-Dichlorphenyl)-4-(2-pyridyl)-1,3-oxazol,
2-(3-Chlor-5-cyanophenyl)-4-(2-pyridyl)-1,3-oxazol,
2-(3-Fluor-5-cyanophenyl)-4-(2-pyridyl)-1,3-oxazol,
2-(3-Chlor-5-fluorphenyl)-4-(2-pyridyl)-1,3-oxazol,
2-(3-Cyanophenyl)-4-(5-chlorpyrid-2-yl)-1,3-oxazol,
2-(3-Cyanophenyl)-4-(5-fluorpyrid-2-yl)-1,3-oxazol,
2-(3-Cyano-5-fluorphenyl)-4-(5-fluorpyrid-2-yl)-1,3-oxazol,
2-(3-Cyanophenyl)-4-(3-fluorpyrid-2-yl)-1,3-oxazol,
2-(3,5-Dimethoxyphenyl)-4-(5-fluorpyrid-2-yl)-1,3-oxazol,
2-(3-Cyanophenyl)-4-(5-methoxypyrid-2-yl)-1,3-oxazol,
2-(3-Cyanophenyl)-4-(3-chlor-5-trifluormethylpyrid-2-yl)-1,3-oxazol,
2-(5-Chlor-2-methoxyphenyl)-4-(2-pyridyl)-1,3-oxazol,
2-(2-Chlor-5-methylthiophenyl)-4-(2-pyridyl)-1,3-oxazol,
2-(2-Brom-5-methoxyphenyl)-4-(2-pyridyl)-1,3-oxazol,
2-(2,5,6-Trifluorphenyl)-4-(2-pyridyl)-1,3-oxazol,
2-[3-Chlorphenyl]-4-[pyridin-2-yl]-1,3-oxazol,
2-(2,5,6-Trifluorphenyl)-4-(2-pyridyl)-1,3-oxazol, 2-(3-Nitrophenyl)-4-(2-pyridyl)-1,3-oxazol oder
2-(3-Bromphenyl)-4-(2-pyridyl)-1,3-oxazol;
unter der Maßgabe, dass die Verbindung nicht 3-(2-Pyridyl)-5-(2-chlorphenyl)-1,2,4-oxadiazol ist, zur Herstellung eines Arzneimittels für die Behandlung von Krankheiten, die mit metabotropen Glutamatrezeptoren in Verbindung stehen.

9. Verwendung nach Anspruch 8, wobei Ar² mit einer oder mehreren Gruppierungen, ausgewählt aus der Gruppe, bestehend aus CF₃, Cl, F, Br, CH₃, CN und SCH₃, substituiert ist.

10. Verwendung nach Anspruch 8, wobei Ar¹ mit einer oder mehreren Gruppierungen, ausgewählt aus der Gruppe, bestehend aus CF₃, F und Cl, substituiert ist.

11. Verwendung nach Anspruch 8, wobei die Krankheit, die mit metabotropen Glutamatrezeptoren in Verbindung steht, eine neurologische Krankheit oder Störung ist.

12. Verwendung nach Anspruch 8, wobei die Krankheit, die mit metabotropen Glutamatrezeptoren in Verbindung steht, eine psychiatrische Krankheit ist.

13. Verwendung nach Anspruch 8, wobei die Krankheit oder Störung ausgewählt ist aus der Gruppe, bestehend aus Schlaganfall, Kopftrauma, Anoxie-bedingter Schädigung, Ischämie-bedingter Schädigung, Hypoglykämie, Epilepsie, Schmerz, Migräne, Morbus Parkinson, Dementia senilis, Chorea Huntington und Morbus Alzheimer.

14. Verwendung nach Anspruch 8, wobei die Krankheit oder Störung ausgewählt ist aus der Gruppe, bestehend aus Schizophrenie und Depression.

## Revendications

1. Composé sélectionné parmi :
a) un composé répondant à la formule II : 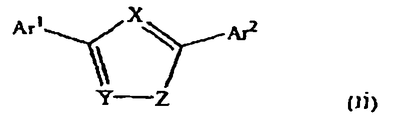 dans laquelle X et Y représentent N,
Z représente O ;
Ar¹ représente un 2-pyridyle et Ar² un phényle, au moins un des groupes Ar¹ et Ar² étant substitué par un ou plusieurs groupes sélectionnés parmi le groupe constitué de -F, -Cl, -Br, -I, -SR, -SOR, -SO₂R, -SO₂NRR', -OCOR, -OCONRR', -NRCOR', -NRCO₂R', -CN, -CO₂R, -CONRR', -C(O)R, -CH(OR)R', -CH₂(OR) et -R ;
dans lesquels R ou R' est sélectionné parmi le groupe constitué de H, CF₃, alkyle en C₁ à C₁₀, cycloalkyle, alkyl-aryle, hétérocycloalkyle, aryle et où R et R' peuvent être combinés pour former un cycle,
à la condition que le composé ne soit ni le 3-(2-pyridyl)-5-(2-chlorophényl)-1,2,4-oxadiazole, ni le 3-(2-pyridyl)-5-phényl-1,2,4-oxadiazole, ni le 3-(2-pyridyl)-5-[3-(trifluorométhyl)phényl]-1,2,4-oxadiazole; ou
b) le 3-(2-pyridyl)-5-[3-(trifluorométhoxy)phényl]-1,2,4-oxadiazole,
le 3-(2-pyridyl)-5-(2,3-difluorophényl)-1,2,4-oxadiazole,
le 3-(2-pyridyl)-5-(3,5-diméthoxyphényl)-1,2,4-oxadiazole,
le 3-(5-fluoropyrid-2-yl)-5-(3,5-diméthoxyphényl)-1,2,4-oxadiazole,
le 3-(5-méthoxypyrid-2-yl)-5-(3-cyanophényl)-1,2,4-oxadiazole,
le 3-(2-pyridyl)-5-(3-nitrophényl)-1,2,4-oxadiazole,
le 2-[3-chlorophényl]-4-[pyridin-2-yl]-1,3-oxazole,
le 2-(3,5-dichlorophényl)-4-(2-pyridyl)-1,3-oxazole,
le 2-(3-chlorophényl)-4-(2-pyridyl)-1,3-oxazole,
le 2-(3-méthoxyphényl)-4-(2-pyridyl)-1,3-oxazole,
le 2-(2-chlorophényl)-4-(2-pyridyl)-1,3-oxazole,
le 2-(3-trifluorophényl)-4-(2-pyridyl)-1,3-oxazole,
le 2-(3-méthylphényl)-4-(2-pyridyl)-1,3-oxazole,
le 2-(3-trifluorométhoxyphényl)-4-(2-pyridyl)-1,3-oxazole,
le 2-(2,3-difluorophényl)-4-(2-pyridyl)-1,3-oxazole,
le 2-(2,5-difluorophényl)-4-(2-pyridyl)-1,3-oxazole,
le 2-(3,5-difluorophényl)-4-(2-pyridyl)-1,3-oxazole,
le 2-(3-cyanophényl)-4-(2-pyridyl)-1,3-oxazole,
le 2-(3,5-diméthoxyphényl)-4-(2-pyridyl)-1,3-oxazole,
le 2-(2,3-dichlorophényl)-4-(2-pyridyl)-1,3-oxazole,
le 2-(3-chloro-5-cyanophényl)-4-(2-pyridyl)-1,3-oxazole,
le 2-(3-fluoro-5-cyanophényl)-4-(2-pyridyl)-1,3-oxazole,
le 2-(3-chloro-5-fluorophényl)-4-(2-pyridyl)-1,3-oxazole,
le 2-(3-cyanophényl)-4-(5-chloropyrid-2-yl)-1,3-oxazole,
le 2-(3-cyanophényl)-4-(5-fluoropyrid-2-yl)-1,3-oxazole,
le 2-(3-cyano-5-fluorophényl)-4-(5-fluoropyrid-2-yl)-1,3-oxazole,
le 2-(3-cyanophényl)-4-(3-fluoropyrid-2-yl)-1,3-oxazole,
le 2-(3,5-diméthoxyphényl)-4-(5-fluoropyrid-2-yl)-1,3-oxazole,
le 2-(3-cyanophényl)-4-(5-méthoxypyrid-2-yl)-1,3-oxazole,
le 2-(3-cyanophényl)-4-(3-chloro-5-trifluorométhylpyrid-2-yl)-1,3-oxazole,
le 2-(5-chloro-2-méthoxyphényl)-4-(2-pyridyl)-1,3-oxazole,
le 2-(2-chloro-5-méthylthiophényl)-4-(2-pyridyl)-1,3-oxazole,
le 2-(2-bromo-5-méthoxyphényl)-4-(2-pyridyl)-1,3-oxazole,
le 2-(2,5,6-trifluorophényl)-4-(2-pyridyl)-1,3-oxazole,
le 2-[3-chlorophényl]-4-[pyridin-2-yl]-1,3-oxazole,
le 2-(2,5,6-trifluorophényl)-4-(2-pyridyl)-1,3-oxazole,
le 2-(3-nitrophényl)-4-(2-pyridyl)-1,3-oxazole,
le 2-(3-bromophényl)-4-(2-pyridyl)-1,3-oxazole,
ou
un sel pharmaceutiquement acceptable de ceux-ci.

2. Composé selon la revendication 1, dans lequel Ar² est substitué par un ou plusieurs groupes sélectionnés parmi le groupe constitué de CF₃, Cl, F, Br, CH₃, SCH₃ et CN.

3. Composé selon la revendication 1, dans lequel Ar¹ est substitué par un ou plusieurs groupes sélectionnés parmi le groupe constitué de CF₃, F et Cl.

4. Composé selon la revendication 1, ledit composé étant sélectionné parmi le groupe constitué du 3-(2-pyridyl)-5-(3,5-dichlorophényl)-1,2,4-oxadiazole, 3-(2-pyridyl)-5-(3-chlorophényl)-1,2,4-oxadiazole, 3-(2-pyridyl)-5-(3-méthylphényl)-1,2,4-oxadiazole, 3-(2-pyridyl)-5-(2,5-difluorophényl)-1,2,4-oxadiazole, 3-(2-pyridyl)-5-(3,5-difluorophényl)-1,2,4-oxadiazole, 3-(2-pyridyl)-5-(3-cyanophényl)-1,2,4-oxadiazole, 3-(2-pyridyl)-5-(2,3-dichlorophényl)-1,2,4-oxadiazole, 3-(2-pyridyl)-5-(3-chloro-5-cyanophényl)-1,2,4-oxadiazole, 3-(2-pyridyl)-5-(3-fluoro-5-cyanophényl)-1,2,4-oxadiazole, 3-(2-pyridyl)-5-(3-chloro-5-fluorophényl)-1,2,4-oxadiazole, 3-(5-chloropyrid-2-yl)-5-(3-cyanophényl)-1,2,4-oxadiazole, 3-(5-fluoropyrid-2-yl)-5-(3-cyanophényl)-1,2,4-oxadiazole, 3-(5-fluoropyrid-2-yl)-5-(3-cyano-5-fluorophényl)-1,2,4-oxadiazole, 3-(3-fluoropyrid-2-yl)-5-(3-cyanophényl)-1,2,4-oxadiazole, 3-(3-chloro-5-trifluorométhylpyrid-2-yl)-5-(3-cyanophényl)-1,2,4-oxadiazole, 3-(2-pyridyl)-5-(5-chloro-2-méthoxyphényl)-1,2,4-oxadiazole, 3-(2-pyridyl)-5-(2-chloro-5-méthylthiophényl)-1,2,4-oxadiazole, 3-(2-pyridyl)-5-(2,5,6-trifluorophényl)-1,2,4-oxadiazole, 3-(2-pyridyl)-5-(2,5,6-trifluorophényl)-1,2,4-oxadiazole, 3-(2-pyridyl)-5-(3-bromophényl)-1,2,4-oxadiazole et les sels pharmaceutiquement acceptables de ceux-ci.

5. Composition pharmaceutique comprenant un composé sélectionné parmi :
a) un composé répondant à la formule II : 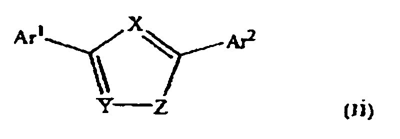 dans laquelle X et Y représentent N,
Z représente O ;
Ar¹ représente un 2-pyridyle et Ar² un phényle, au moins un des groupes Ar¹ et Ar² étant substitué par un ou plusieurs groupes sélectionnés parmi le groupe constitué de -F, -Cl, -Br, -I, -SR, -SOR, -SO₂R, -SO₂NRR' , -OCOR, -OCONRR', -NRCOR', -NRCO₂R', -CN, -CO₂R, -CONRR', -C(O)R, -CH(OR)R', -CH₂(OR) et -R ;
dans lesquels R ou R' est sélectionné parmi le groupe constitué de H, CF₃, alkyle en C₁ à C₁₀, cycloalkyle, alkyl-aryle, hétérocycloalkyle, aryle et où R et R' peuvent être combinés pour former un cycle,
à la condition que le composé ne soit ni le 3-(2-pyridyl)-5-(2-chlorophényl)-1,2,4-oxadiazole ni le3-(2-pyridyl)-5-[3-(trifluorométhyl)phényl]-1,2,4-oxadiazole ; ou
b) le 3-(2-pyridyl)-5-[3-(trifluorométhoxy)phényl]-1,2,4-oxadiazole,
le 3-(2-pyridyl)-5-(2,3-difluorophényl)-1,2,4-oxadiazole,
le 3-(2-pyridyl)-5-(3,5-diméthoxyphényl)-1,2,4-oxadiazole,
le 3-(5-fluoropyrid-2-yl)-5-(3,5-diméthoxyphényl)-1,2,4-oxadiazole,
le 3-(5-méthoxypyrid-2-yl)-5-(3-cyanophényl)-1,2,4-oxadiazole,
le 3-(2-pyridyl)-5-(3-nitrophényl)-1,2,4-oxadiazole,
le 2-[3-chlorophényl]-4-[pyridin-2-yl]-1,3-oxazole,
le 2-(3,5-dichlorophényl)-4-(2-pyridyl)-1,3-oxazole,
le 2-(3-chlorophényl)-4-(2-pyridyl)-1,3-oxazole,
le 2-(3-méthoxyphényl)-4-(2-pyridyl)-1,3-oxazole,
le 2-(2-chlorophényl)-4-(2-pyridyl)-1,3-oxazole,
le 2-(3-trifluorophényl)-4-(2-pyridyl)-1,3-oxazole,
le 2-(3-méthylphényl)-4-(2-pyridyl)-1,3-oxazole,
le 2-(3-trifluorométhoxyphényl)-4-(2-pyridyl)-1,3-oxazole,
le 2-(2,3-difluorophényl)-4-(2-pyridyl)-1,3-oxazole,
le 2-(2,5-difluorophényl)-4-(2-pyridyl)-1,3-oxazole,
le 2-(3,5-difluorophényl)-4-(2-pyridyl)-1,3-oxazole,
le 2-(3-cyanophényl)-9-(2-pyridyl)-1,3-oxazole,
le 2-(3,5-diméthoxyphényl)-4-(2-pyridyl)-1,3-oxazole,
le 2-(2,3-dichlorophényl)-4-(2-pyridyl)-1,3-oxazole,
le 2-(3-chloro-5-cyanophényl)-4-(2-pyridyl)-1,3-oxazole,
le 2-(3-fluoro-5-cyanophényl)-4-(2-pyridyl)-1,3-oxazole,
le 2-(3-chloro-5-fluorophényl)-4-(2-pyridyl)-1,3-oxazole,
le 2-(3-cyanophényl)-4-(5-chloropyrid-2-yl)-1,3-oxazole,
le 2-(3-cyanophényl)-4-(5-fluoropyrid-2-yl)-1,3-oxazole,
le 2-(3-cyano-5-fluorophényl)-4-(5-fluoropyrid-2-yl)-1,3-oxazole,
le 2-(3-cyanophényl)-4-(3-fluoropyrid-2-yl)-1,3-oxazole,
le 2-(3,5-diméthoxyphényl)-4-(5-fluoropyrid-2-yl)-1,3-oxazole,
le 2-(3-cyanophényl)-4-(5-méthoxypyrid-2-yl)-1,3-oxazole,
le 2-(3-cyanophényl)-4-(3-chloro-5-trifluorométhylpyrid-2-yl)-1,3-oxazole,
le 2-(5-chloro-2-méthoxyphényl)-4-(2-pyridyl)-1,3-oxazole,
le 2-(2-chloro-5-méthylthiophényl)-4-(2-pyridyl)-1,3-oxazole,
le 2-(2-bromo-5-méthoxyphényl)-4-(2-pyridyl)-1,3-oxazole,
le 2-(2,5,6-trifluorophényl)-4-(2-pyridyl)-1,3-oxazole,
le 2-[3-chlorophényl]-4-[pyridin-2-yl]-1,3-oxazole,
le 2-(2,5,6-trifluorophényl)-4-(2-pyridyl)-1,3-oxazole,
le 2-(3-nitrophényl)-4-(2-pyridyl)-1,3-oxazole,
le 2-(3-bromophényl)-4-(2-pyridyl)-1,3-oxazole, ou
un sel pharmaceutiquement acceptable de ceux-ci ;
et un excipient pharmaceutiquement acceptable.

6. Composition selon la revendication 5, dans laquelle Ar² est substitué par un ou plusieurs groupes sélectionnés parmi le groupe constitué de CF₃, Cl, F, Br, CH₃, CN et SCH₃.

7. Composition selon la revendication 5, dans laquelle Ar¹ est substitué par un ou plusieurs groupes sélectionnés parmi le groupe constitué de CF₃, F et Cl.

8. Utilisation d'une quantité pharmaceutiquement acceptable d'un composé sélectionné parmi :
a) un composé répondant à la formule II : 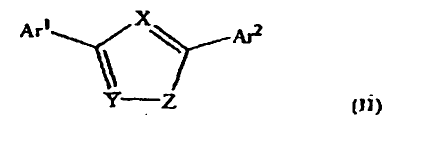 dans laquelle X et Y représentent N,
Z représente O ;
Ar¹ représente un 2-pyridyle et Ar² un phényle, au moins un des groupes Ar¹ et Ar² étant substitué par un ou plusieurs groupes sélectionnés parmi le groupe constitué de -F, -Cl, -Br, -I, -SR, -SOR, -SO₂R, - SO₂NRR', -OCOR, -OCONRR', -NRCOR', -NRCO₂R', -CN, -CO₂R, - CONRR', -C(O)R, -CH(OR)R', -CH₂(OR) et -R ;
dans lesquels R ou R' est sélectionné parmi le groupe constitué de H, CF₃, alkyle en C₁ à C₁₀, cycloalkyle, alkyl-aryle, hétérocycloalkyle, aryle et où R et R' peuvent être combinés pour former un cycle,
à la condition que le composé ne soit ni le 3-(2-pyridyl)-5-(2-chlorophényl)-1,2,4-oxadiazole ni le3-(2-pyridyl)-5-[3-(trifluorométhyl)phényl]-1,2,4-oxadiazole ; ou
b) le 3-(2-pyridyl)-5-[3-(trifluorométhoxy)phényl]-1,2,4-oxadiazole,
le 3-(2-pyridyl)-5-(2,3-difluorophényl)-1,2,4-oxadiazole,
le 3-(2-pyridyl)-5-(3,5-diméthoxyphényl)-1,2,4-oxadiazole,
le 3-(5-fluoropyrid-2-yl)-5-(3,5-diméthoxyphényl)-1,2,4-oxadiazole,
le 3-(5-méthoxypyrid-2-yl)-5-(3-cyanophényl)-1,2,4-oxadiazole,
le 3-(2-pyridyl)-5-(3-nitrophényl)-1,2,4-oxadiazole,
le 2-[3-chlorophényl]-4-[pyridin-2-yl]-1,3-oxazole,
le 2-(3,5-dichlorophényl)-4-(2-pyridyl)-1,3-oxazole,
le 2-(3-chlorophényl)-4-(2-pyridyl)-1,3-oxazole,
le 2-(3-méthoxyphényl)-4-(2-pyridyl)-1,3-oxazole,
le 2-(2-chlorophényl)-4-(2-pyridyl)-1,3-oxazole,
le 2-(3-trifluorophényl)-4-(2-pyridyl)-1,3-oxazole,
le 2-(3-méthylphényl)-4-(2-pyridyl)-1,3-oxazole,
le 2-(3-trifluorométhoxyphényl)-4-(2-pyridyl)-1,3-oxazole,
le 2-(2,3-difluorophényl)-4-(2-pyridyl)-1,3-oxazole,
le 2-(2,5-difluorophényl)-4-(2-pyridyl)-1,3-oxazole,
le 2-(3,5-difluorophényl)-4-(2-pyridyl)-1,3-oxazole,
le 2-(3-cyanophényl)-4-(2-pyridyl)-1,3-oxazole,
le 2-(3,5-diméthoxyphényl)-4-(2-pyridyl)-1,3-oxazole,
le 2-(2,3-dichlorophényl)-4-(2-pyridyl)-1,3-oxazole,
le 2-(3-chloro-5-cyanophényl)-4-(2-pyridyl)-1,3-oxazole,
le 2-(3-fluoro-5-cyanophényl)-4-(2-pyridyl)-1,3-oxazole,
le 2-(3-chloro-5-fluorophényl)-4-(2-pyridyl)-1,3-oxazole,
le 2-(3-cyanophényl)-4-(5-chloropyrid-2-yl)-1,3-oxazole,
le 2-(3-cyanophényl)-4-(5-fluoropyrid-2-yl)-1,3-oxazole,
le 2-(3-cyano-5-fluorophényl)-4-(5-fluoropyrid-2-yl)-1,3-oxazole,
le 2-(3-cyanophényl)-4-(3-fluoropyrid-2-yl)-1,3-oxazole,
le 2-(3,5-diméthoxyphényl)-4-(5-fluoropyrid-2-yl)-1,3-oxazole,
le 2-(3-cyanophényl)-4-(5-méthoxypyrid-2-yl)-1,3-oxazole,
le 2-(3-cyanophényl)-4-(3-chloro-5-trifluorométhylpyrid-2-yl)-1,3-oxazole,
le 2-(5-chloro-2-méthoxyphényl)-4-(2-pyridyl)-1,3-oxazole,
le 2-(2-chloro-5-méthylthiophényl)-4-(2-pyridyl)-1,3-oxazole,
le 2-(2-bromo-5-méthoxyphényl)-4-(2-pyridyl)-1,3-oxazole,
le 2-(2,5,6-trifluorophényl)-4-(2-pyridyl)-1,3-oxazole,
le 2-[3-chlorophényl]-4-[pyridin-2-yl]-1,3-oxazole,
le 2-(2,5,6-trifluorophényl)-4-(2-pyridyl)-1,3-oxazole,
le 2-(3-nitrophényl)-4-(2-pyridyl)-1,3-oxazole, ou
le 2-(3-bromophényl)-4-(2-pyridyl)-1,3-oxazole ;
à la condition que le composé ne soit pas le 3-(2-pyridyl)-5-(2-chlorophényl)-1,2,4-oxadiazole, pour la préparation d'un médicament utilisé dans le traitement des maladies associées aux récepteurs du glutamate métabotrope.

9. Utilisation selon la revendication 8, dans laquelle Ar² est substitué par un ou plusieurs groupes sélectionnés parmi le groupe constitué de CF₃, Cl, F, Br, CH₃, CN et SCH₃.

10. Utilisation selon la revendication 8, dans laquelle Ar¹ est substitué par un ou plusieurs groupes sélectionnés parmi le groupe constitué de CF₃, F et Cl.

11. Utilisation selon la revendication 8, dans laquelle la maladie associée aux récepteurs du glutamate métabotrope est une maladie ou un trouble neurologique.

12. Utilisation selon la revendication 8, dans laquelle la maladie associée aux récepteurs du glutamate métabotrope est une maladie psychiatrique.

13. Utilisation selon la revendication 8, dans laquelle la maladie ou le trouble fait partie du groupe consistant en un accident vasculaire cérébral, un traumatisme crânien, un accident anoxique, un accident ischémique, l'hypoglycémie, l'épilepsie, la douleur, la migraine, la maladie de Parkinson, la démence sénile, la chorée de Huntington et la maladie d'Alzheimer.

14. Utilisation selon la revendication 8, dans laquelle la maladie ou le trouble fait partie du groupe consistant en la schizophrénie et la dépression.
